# EUROPEAN PATENT APPLICATION

(11) **EP 2 332 562 A2**
(43) Date of publication of application: **15.06.2011**
(21) Application number: 09009585.2
(22) Date of filing: 23.07.2009
(51) Int. Cl.: A61K 38/16

(54) **Therapeutic agents for the treatment of tuberculosis, especially pulmonary tuberculosis**

(30) Priority: 09.04.2009 EP 09005235
(71) Applicant: Universität zu Köln, 50923 Köln (DE)
(72) Inventor: Rybniker, Jan, Dr., 50937 Köln (DE); Hartmann, Pia, Dr., 50937 Köln (DE)
(74) Representative: Gesthuysen, von Rohr & Eggert

(57) **Abstract**

The present invention relates to the use of a pharmacologically active agent being able to inhibit or at least to reduce the expression and/or activity of a (poly-)peptide and/or a protein of a tuberculosis inducing germ, especially the genus *Mycobacterium,* for producing a medicament or pharmaceutical for the prophylactic and/or therapeutic (curative) treatment of tuberculosis, especially pulmonary tuberculosis, wherein the (poly-)peptide and/or protein is associated with or related to the reproduction, especially cell division, of the tuberculosis inducing germ.

## Description

The present invention relates to a therapeutic concept for the treatment of tuberculosis, especially pulmonary tuberculosis, especially a therapeutic concept on the basis of specific WhiB-like proteins, which influence the viability of tuberculosis pathogens, especially bacterial species of the genus *Mycobacterium*.

Especially, the present invention refers to the use of a pharmacologically active agent and/or a pharmacologically active substance being able to inhibit or at least to reduce the expression and/or activity of a (poly-)peptide and/or a protein of a tuberculosis germ for the prophylactic and/or therapeutic (curative) treatment of tuberculosis.

The present invention also refers to the use of at least one pharmacologically active agent, especially at least one inhibitor and/or repressor, for producing a medicament or pharmaceutical for the prophylactic and/or therapeutic (i.e. curative) treatment of tuberculosis.

The present invention also refers to the use of a gene encoding for a (poly-)peptide and/or a protein of a tuberculosis inducing germ for finding and/or providing a pharmaceutically active agent for the prophylactic and/or therapeutic (i.e. curative) treatment of tuberculosis.

The present invention also refers to methods of identifying and/or retrieving a pharmacologically active agent being able to inhibit or at least to reduce the expression and/or activity of a specific (poly-)peptide and/or a protein of a tuberculosis inducing germ.

Moreover, the present invention also refers to a method of improving the pharmacological properties of a pharmacologically active agent.

Furthermore, the present invention also refers to a method of modification of the pharmacologically active agent.

The present invention also refers to a pharmaceutically active agent as such.

In addition, the present invention also refers to a pharmaceutical composition which comprises an efficient amount of at least one pharmacologically active agent being able to inhibit or at least to reduce the expression and/or activity of a peptide and/or a protein of a tuberculosis inducing germ.

Finally, the present invention refers to a method of treating a human suffering from tuberculosis, especially pulmonary tuberculosis.

Tuberculosis, which is commonly also abbreviated as TB for tubercle bacillus or tuberculosis, is a common and often deadly infectious disease caused by mycobacteria, like *Mycobacterium tuberculosis.* Tuberculosis usually attacks the lungs - in such case denoted as pulmonary tuberculosis - but can also affect the central nervous system, the lymphatic system, the circulatory system, the genitourinary system, the gastrointestinal system, bones, joints and even the skin.

The primary cause of tuberculosis, *Mycobacterium tuberculosis,* is an aerobic bacterium that divides every 16 to 20 hours, i.e. with an extremely slow rate if compared with other bacteria. *Mycobacterium tuberculosis* is classified as a Gram-positive bacterium. *Mycobacterium tuberculosis* is a small rod-like bacillus that can withstand weak disinfectants and survive in a dry state for weeks. In nature, the bacterium can grow only within the cells of a host organism, but *Mycobacterium tuberculosis* can be cultured in vitro. Further bacteria species being of high relevance in view of the development and induction of tuberculosis and tuberculosis-like diseases are e.g. *Mycobacterium bovis, Mycobacterium africanum, Mycobacterium canetti* and *Mycobacterium microti.* Other known pathogenic mycobacteria include *Mycobacterium leprae, Mycobacterium avium* and *Mycobacterium kansasii.* The last two are part of the nontuberculous mycobacteria group. Nontuberculous mycobacteria cause neither tuberculosis nor leprosy, but they do cause pulmonary diseases resembling tuberculosis.

Further mycobacterium species, like *Mycobacterium smegmatis,* are known in the prior art which are not directly linked with tuberculosis diseases but which, however, represent well-established model systems allowing a direct correlation to pathogenic germs, like *Mycobacterium tuberculosis,* especially due to their comparable physiology.

The classic symptoms of tuberculosis are chronic cough with blood-tinged sputum and chest pain. Systemic symptoms include fever, chills, night sweats, appetite loss, weight loss, pallor and often a tendency to fatigue very easily. Infection of other organs causes a wide range of symptoms. When the disease becomes active, 75 % of the cases are pulmonary tuberculosis. In the other 25 % of active cases, the infection moves from the lungs, causing other kinds of tuberculosis, collectively denoted extrapulmonary tuberculosis. This occurs more commonly in immunosuppressed persons and young children. Extrapulmonary infection sites include the pleura in tuberculosis pleurisy, the central nervous system in meningitis, the lymphatic system in scrofula of the neck, the genitourinary system in urogenital tuberculosis, and bones and joints in Pott's disease of the spine. An especially serious form is disseminated tuberculosis, more commonly known as miliary tuberculosis.

Tuberculosis is generally spread through the air, when people who have the active disease cough, sneeze or spit. When people suffering from active pulmonary tuberculosis cough, sneeze, speak, or spit, they expel infectious aerosol droplets. A single sneeze can release up to 40,000 droplets. Each one of these droplets may transmit the disease, since the infectious dose of tuberculosis is very low and the inhalation of just a single bacterium can cause a new infection. People with prolonged, frequent or intense contact are at particularly high risk of becoming infected, with an estimated 22 % infection rate. A person with active but untreated tuberculosis can infect 10 to 15 other people per year.

One third of the world's current population - nearly 2 billion people - has been infected with *Mycobacterium tuberculosis,* and new infections occur at a rate of one per second. About one in ten of these latent infections will eventually progress to active disease, which, if left untreated, kills more than half of its victims. The absolute number of people in the general population who become sick with tuberculosis each year is still increasing. In 2004, mortality and morbidity statistics included 14.6 million chronic active cases, 8.9 million new cases, and 1.6 million deaths, mostly in developing countries. If untreated, the death rate for active tuberculosis cases is more than 50 %.

Tuberculosis is classified as one of the granulomatous inflammatory conditions. Macrophages, T lymphocytes, B lymphocytes and fibroblasts are among the cells that aggregate to form a granuloma, with lymphocytes surrounding the infected macrophages. The granuloma functions not only to prevent dissemination of the mycobacteria, but also provides a local environment for communication of cells of the immune system. Importantly, bacteria are not always eliminated within the granuloma, but can become dormant, resulting in a latent infection. Another feature of the granuloma of human tuberculosis is the development of cell death, also called necrosis, in the center of tubercles. To the naked eye this has the texture of soft white cheese and was termed caseous necrosis.

If tuberculosis bacteria gain entry to the bloodstream from an area of damaged tissue, they spread through the body and set up many foci of infection, all appearing as tiny white tubercles in the tissues. This severe form of tuberculosis disease is most common in infants and in elderly and is called miliary tuberculosis. Patients with this disseminated tuberculosis have a fatality rate of approximately 20 %, even with intensive treatment.

To sum up, tuberculosis as a clinical disease or infection can be caused by germs and/or pathogens belonging to the so called *"Mycobacterium tuberculosis* complex". Typical members of this complex are *Mycobacterium tuberculosis, Mycobacterium bovis, Mycobacterium africanum, Mycobacterium canetti* and *Mycobacterium microti.* Thus, all tuberculosis germs belong to the genus *Mycobacterium.* Other mycobacteria than those belonging to the *Mycobacterium tuberculosis* complex are summarized as "mycobacteria other than tuberculosis" (MOTT) or "nontuberculosis mycobacteria," (NTM). On the whole, about 135 species are known, 50 of which are pathogenic to humans. To these species belong *Mycobacterium avium, Mycobacterium marinum, Mycobacterium ulcerans* and *Mycobacterium kansasii,* however, these germs do not induce tuberculosis but may be associated with other diseases. According to the prior art, treatment for tuberculosis generally uses antibiotics to kill the pathogenic bacteria. The most commonly used antibiotics are rifampicin, isoniazide, ethambutol and pyrazinamide. However, instead of the short course of antibiotics typically used to cure other bacterial infections, tuberculosis requires much longer periods of treatment, i.e. around 6 to 12 months, to entirely eliminate mycobacteria from the body. Active tuberculosis disease is best treated with combinations of several antibiotics in order to reduce the risk of the bacteria developing antibiotic resistance. However, the use of the above antibiotics is linked to the problem of increased side-effects, comprising associated liver injury, polyneuropathy, inflammations of the optic nerve or damage of kidneys and inner ear.

Another decisive problem concerning the treatment of tuberculosis with prior art methods is to be seen in growing resistance of the pathogenic germs in view of the used antibiotics. Primary resistance occurs in persons who are infected with a resistant strain of tuberculosis. A patient with fully-susceptible tuberculosis develops secondary resistance, i.e. acquired resistance, during tuberculosis therapy because of inadequate treatment, not taking the prescribed regimen appropriately, or using low quality medication.

On the whole, drug-resistant and especially multi-drug-resistant tuberculosis is a growing public health issue of concern. Multi-drug-resistant tuberculosis is defined as the resistance to the two most effective first-line tuberculosis drugs: rifampicin and isoniazid. Extensively drug-resistant tuberculosis is also resistant to three or more of the six classes of second-line drugs.

Another decisive challenge of the treatment of tuberculosis has to be seen in the fact that it is explicitly difficult to provide pharmaceutical agents being able to attain the site of pharmacologic action. Especially, the uptake and incorporation of pharmaceutical agents into the pathogens are often difficult, especially since pharmaceutical agents have to penetrate through the rigid bacterial wall of the tuberculosis inducing germ.

Moreover, vaccination may form another aspect in treatment and prevention, respectively, of tuberculosis. However, although a certain protective efficacy of vaccination for preventing serious forms of tuberculosis is given in children, its protective efficacy for preventing pulmonary tuberculosis in adolescents and adults is variable and not satisfying. In this context, it has been shown that vaccination is unreliable against adult pulmonary tuberculosis, which accounts for most of the disease burden worldwide.

To sum up, it has to be pointed out that tuberculosis represents a growing and very serious disease, the treatment of which is difficult and requires long-term therapy with multiple antibiotics often linked with serious side-effects and deleterious impact on the health of the patient. In addition, antibiotic resistance is a growing problem, especially in extensively multi-drug-resistant tuberculosis. Moreover, vaccination does not lead to strong success in the prevention of this disease. Currently, there are more cases of tuberculosis on the planet than at any other time in history.

Thus, there is an urgent need for providing new therapeutic approaches being effective with respect to the treatment of tuberculosis. The dramatic side-effects and the increasing incidence of multiple resistances require the development of entirely new classes of substances and mechanisms to fight tuberculosis.

Therefore, especially in the light of the above-mentioned medical background, it is an object of the present invention to provide a new and effective concept or approach with respect to the prophylactic and/or therapeutic (i.e. curative) treatment of tuberculosis, which is to overcome or at least to diminish the aforementioned disadvantages linked to the prior art methods.

Especially, it is an object of the present invention to provide specific pharmacologically effective substances and agents exhibiting a high effectiveness in the field of the treatment of tuberculosis, especially with respect to *Mycobacterium* strains, as well as to provide effective methods and processes for identifying and/or providing such substances or agents.

Furthermore, it is another object of the present invention to provide tuberculosis antagonizing agents having reduced side-effects if compared to the prior art agents, thereby at the same time being highly effective at the site of pharmacological action, i.e. having improved properties with respect to the uptake by and incorporation into the pathologic germ.

Additionally, it is another object of the present invention to provide therapeutic agents having an improved therapeutic effect that prevent all forms of tuberculosis including drug-resistant strains in all age groups and also among people with accompanying immunosuppressive diseases.

Moreover, it is an object of the present invention to provide methods for the identification of new agents exhibiting a high pharmacological effectiveness against tuberculosis, especially against strains of the genus *Mycobacterium.*

Surprisingly, applicant has found out that the aforedescribed objects can be solved, *inter alia,* by the subject-matter of Claim 1; further, especially advantageous embodiments are the subject-matter of the respective dependent claims.

Furthermore, according to another aspect of the present invention, the present invention refers to the use of at least one active agent, especially at least one inhibitor and/or repressor, for producing a medicament or pharmaceutical for the prophylactic and/or therapeutic (i.e. curative) treatment of tuberculosis according to Claim 7.

Moreover, according to another aspect of the present invention, the present invention refers to the use of a gene encoding for a (poly-)peptide and/or protein of a tuberculosis inducing germ for finding and/or providing a pharmaceutical composition especially for the prophylactic and/or therapeutic (i.e. curative) treatment of tuberculosis according to Claim 8.

Furthermore, according to a further aspect of the present invention, the present invention refers to methods of identifying and/or retrieving a pharmacologically active agent being able to inhibit or at least to reduce the expression and/or activity of a (poly-)peptide and/or a protein of a tuberculosis inducing germ according to Claim 9 and 10.

According to another aspect of the present invention, the present invention refers to a method of improving the pharmacological properties of a pharmacologically active agent as identified by the above methods according to Claim 11.

The present invention also refers, according to another aspect of the present invention and according to Claim 12, to a method of modification of a pharmacologically active agent identified or improved by the inventive methods.

Moreover, the present invention also refers - according to Claim 13 - to the pharmaceutically active agent as such, which is identified and/or modified by the inventive methods.

Moreover, according to another aspect of the present invention, the present invention refers to a pharmaceutical composition, especially for the prophylactic and/or therapeutic (i.e. curative) treatment of tuberculosis according to Claim 14.

Finally, according to another aspect of the present invention, the present invention refers to a method of treating a human suffering from tuberculosis according to Claim 15.

In the following, it has to be noted that explanations, details, examples etc. given with respect to one aspect of the present invention only shall, of course, also refer to all the other aspects of the present invention, even without explicit mentioning of this fact. Thus, it may well be in the following that specific aspects, explanations, details etc. are only delineated with respect to one specific embodiment only in order to avoid unnecessary repetitions, but they also apply with respect to all other aspects of the present invention.

Applicant now has surprisingly found out a new approach for the treatment of tuberculosis. Especially, applicant has found out that the purposeful pharmacological influence of specific compounds on specific (poly-)peptides and/or proteins of a tuberculosis germ - especially from the genus *Mycobacterium -* leads to a loss of viability of the affected tuberculosis germ. In more detail, applicant has surprisingly found out that an influence on the expression and/or activity in the sense of a down-regulations or decrease of a specific (poly-)peptide and/or protein of the tuberculosis inducing germ results in a significantly decreased viability of the germ, thus representing an effective approach for the treatment of tuberculosis. In this context, the (poly-)peptide and/or protein of the tuberculosis inducing germ, which represents the pharmacological and/or drug target on the level of the gene encoding for it and/or on the level of transcription for translation products, like the protein itself according to the inventive concept, is normally associated with or related to the reproduction, especially cell division, of the tuberculosis inducing germ. Thus, the present invention focuses on the purposeful knock-out of a native (poly-)peptide and/or protein of the tuberculosis inducing germ, which is essential for the reproduction, especially cell division, of the germ. Thus, the purposeful knock-out, inactivation and down-regulation, respectively, of such (poly-)peptide and/or protein results in losing the capability of cell division.

In this context, applicant has found out, for the first time, that the expression of a pharmacologically active WhiB-like protein of mycobacteriophage TM4 (WhiBTM4) leads to a knockout phenotype of the essential WhiB2/WhmD gene of the mycobacterium and/or the respective (poly-)peptide and/or protein encoded by this gene, resulting in hindered septation and cell division with branched and filamentous appearance of mycobacteria, especially *Mycobacterium smegmatis,* subsequently leading to a loss of viability. Furthermore, applicant has shown that WhiBTM4 contains specific pharmacologically relevant motifs forming the basis and - in a manner of speaking - the leitmotif for other effective drugs and agents. For, applicant has shown that cysteine residues, especially four cysteine residues, coordinate iron/sulfur-clusters (Fe-S-Cluster) within the WhiBTM4 protein. Especially, the UV-visible absorption spectra of freshly purified WhiBTM4 shows the presence of a [2Fe-2S]-Cluster which becomes decomposed under atmospheric oxygen. This [2Fe-2S]-Cluster can be reconstituted under anaerobic conditions. Furthermore, applicant has shown the pharmacological relevance of a so-called helix-turn-helix motif of the protein. On the basis of the described knockout phenotype, a method for identifying compounds being effective with respect to the treatment of tuberculosis is provided by the present invention.

Especially, on the basis of the effective WhiBTM4 protein, relevant structures are identified, which are important for the above described effectiveness, thus forming the starting point for the generation of optimized and even more efficient drugs and pharmaceutical agents. For example, the described knockout phenotype enables to screen for WhiBTM4 mutations, which allow mycobacterial growth further specifying the relevant properties of an efficient drug. In this context, applicant has shown that mutations of the Fe-S-complex coordinating amino acids result in wild-type colony morphology and mutations in the N-terminal part of the protein, leading to a brown colony morphology most likely indicating the accumulation of the Fe-S-complex, pointing to the pharmacological relevance of these motifs and/or regions of the protein. On the whole, applicant has found regions of the protein being of high importance with respect to the effectiveness of a potential drug. To sum up, applicant's results show that WhiBTM4 is a functional Fe-S-cluster protein which competes with wild-type WhiB2/WhmD, leading to aberrant growth of the bacteriophage host. On the basis of the pharmacologically relevant regions of WhiBTM4, according to the present invention, also a method is provided according to which optimized drugs and pharmaceutically active agents, especially so-called small molecules, can be developed, having improved properties e.g. with respect to their incorporation into the mycobacterial host.

Therefore, on the whole, the present invention provides, for the first, time a therapeutic approach with respect to the treatment of tuberculosis diseases focusing in a very specific manner on the influence of specific (poly-)peptides and/or proteins of the host bacterium being necessary for cell-division.

Thus, according to a **first** aspect of the present invention, there is provided a use of a pharmacologically active agent being able to inhibit or at least to reduce the expression and/or activity of a (poly-)peptide and/or a protein of a tuberculosis inducing germ, especially of the genus *Mycobacterium,* for producing a medicament or pharmaceutical for the prophylactic and/or therapeutic (i.e. curative) treatment of tuberculosis, especially pulmonary tuberculosis, wherein the peptide and/or protein is associated with or related to the reproduction, especially cell division, of the tuberculosis inducing germ.

The term "tuberculosis inducing germ" as used according to the present invention refers to both pathogenic germs and also to germs being related with tuberculosis, for example in the sense that these germs causes pulmonary diseases resembling tuberculosis, which is the case for example for *Mycobacterium avium* and *Mycobacterium kansasii.* The term "tuberculosis inducing germ" also includes germs being related to tuberculosis in so far as they serve as a model system which can be directly correlated to the pathogenic tuberculosis germs, especially due to their physiological similarity, i.e. this term also comprises germs which do not cause tuberculosis as such as a clinical disease and infection, respectively, but also model germs as such and especially also the aforedescribed MOTT and NTM germs, especially since these germs are principally appropriate as model systems on behalf of the present invention. For this reason, the term "tuberculosis inducing germ" may synonymously also be denoted as "tuberculosis related germ". In this context, *Mycobacterium smegmatis,* which itself does not cause tuberculosis symptoms, can be used as a model system due to the physiological comparability and similarity to pathogenic germs like. This point is of high relevance, especially with respect to the inventive concept of finding and/or modifying pharmacologically active agents being effective with respect to the treatment of tuberculosis in the context of which the aforementioned model system can be used. Thus, on the whole, the term "tuberculosis inducing germ" is to be understood in a very broad manner comprising germs as such, especially of the genus *Mycobacterium,* which relate to tuberculosis in the sense of disease-inducing germs as well as germs being broadly related to the disease, especially in the sense of model systems allowing for transferring experimental findings to pathogenic counter part.

According to the inventive concept, generally all mycobacteria in which the knockout and/or downregulation of the phenotype may be induced due to their physiological properties constitute a potential target for the functional domain(s) of WhiBTM4. Also for this reason, the term "tuberculosis, especially pulmonary tuberculosis" may also be understood synonymously as "mycobacterial diseases including tuberculosis" and the term "tuberculosis inducing germ" synonymously as "mycobacteria, especially *Mycobacterium tuberculosis* complex".

Furthermore, the term "associated with" or "related to" with respect to the (poly-)peptide and/or protein being relevant for the reproduction of the tuberculosis inducing germs is also to be understood in a broad manner and generally refers to natural and/or native proteins of the respective germ normally expressed by the tuberculosis inducing germ and playing a vital role with respect to the reproduction and cell division of the germ, the (poly-)peptides and/or proteins representing the intrinsic drug target of the germ to be treated with respect to the pharmacologically active agent.

According to a specific embodiment, the tuberculosis inducing germ is selected from the group of *Mycobacterium* species, especially *Mycobacterium tuberculosis, Mycobacterium bovis, Mycobacterium africanum, Mycobacterium canetti, Mycobacterium microti, Mycobacterium leprae, Mycobacterium avium* and *Mycobacterium kansasii.* As delineated above, in general all germs of the MOTT and/or the NTM group are applicable as model germs on behalf of the present invention regardless of their pathogenicity due to their specific properties being common for all members of the genus of mycobacteria. For example, *Mycobacterium avium,* which may cause pulmonary infections, may also be used as a model germ even though this is not preferred due to the slow growth of this germ in comparison to *Mycobacterium smegmatis.* According to the present invention, the use of the apathogenic saprophyt as a model germ is preferred since *Mycobacterium smegmatis* has excellent growing properties and can be easily transfected. On the whole, all mycobacteria can be used as model germs and/or target systems on behalf of the present invention in so far as - on the basis of their physiological properties - a knockout and/or inactivation and/or downregulation in the sense of the present invention of the respective peptide and/or protein being associated with or related to the reproduction, especially cell division, of the tuberculosis inducing germ can be induced. On the whole, all germs of the *Mycobacterium tuberculosis* complex and the MOTT and/or the NTM group have in common a rigid constitution of the wall structure and similar physiological properties, which are also responsible for granulomatous inflammations associated with mycobacteria.

The present invention is not limited to the inventive use of the pharmacologically active agent for the treatment of tuberculosis induced by the above-mentioned tuberculosis germs. In fact, the present invention in general refers to the use of the pharmacologically active agent for the treatment of tuberculosis as such in so far as the respective germ to be treated exhibits a (poly-)peptide and/or protein in the sense of the present invention being associated with or related to the reproduction of the germ and being affected by the pharmacologically active agent.

Moreover, in the light of the above given definition, the present invention in general refers to the treatment and/or use also of germs being not pathogenic as such but exhibiting a direct correlation and comparability to pathogenic tuberculosis germs, e.g. due to a directly comparable physiology, especially with respect to the reproduction of the germ. In this context, the present invention directly includes e.g. the germ *Mycobacterium smegmatis* in the sense of a model system with respect to pathogenic tuberculosis germ.

As to the (poly-)peptide and/or protein associated with or related to the reproduction, especially cell division, of the tuberculosis inducing germ, according to a preferred embodiment of the present invention, this (poly-)peptide and/or protein may be associated with or related to the septum formation and/or fragmentation of the tuberculosis inducing germ.

Especially, the (poly-)peptide and/or protein associated with or related to the reproduction, especially cell division, of the tuberculosis inducing germ is a WhiB-like protein, especially a protein of the WhiB-family, preferably WhiB2, more preferably WhiB2 of a species of the genus *Mycobacterium,* even more preferably WhiB2 of *Mycobacterium tuberculosis.*

For, it has been shown, that WhiB2 of *Mycobacterium tuberculosis* plays a decisive role in mycobacterial cell division, thus making WhiB2 an interesting and important drug target.

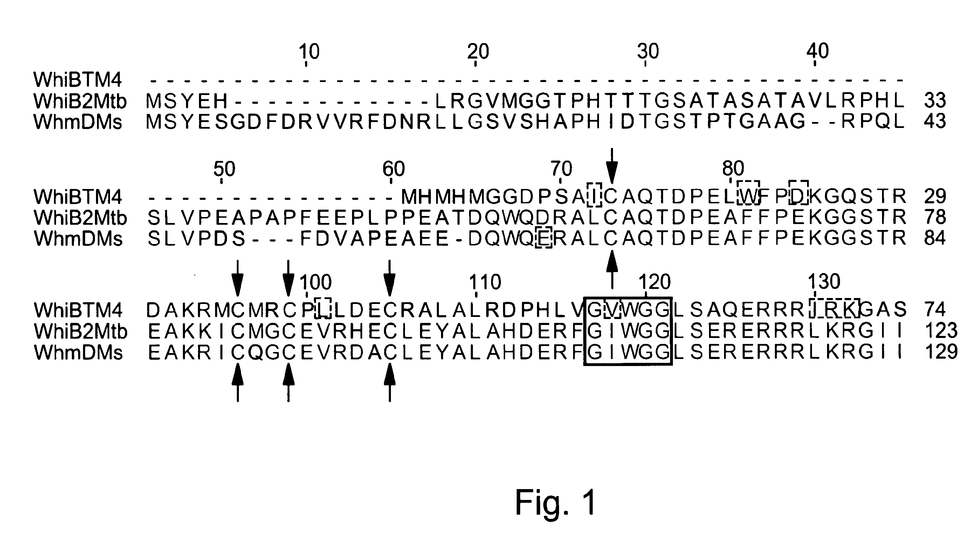

Especially, the WhiB2-protein of *Mycobacterium tuberculosis* is used as the drug target - and this on the level of a influence the gene encoding for this protein and/or respect of transcription and/or translation products - and, thus, as its function as the (poly-)peptide and/or protein being associated with or related to the reproduction, especially cell division, of the tuberculosis inducing germ, has the amino acid sequence as given in Fig. 1 (cf. WhiB2Mtb of Fig. 1), which is given according to the well-known one letter code.

According to the present invention, the (poly-)peptide and/or protein associated with or related to the reproduction, especially cell division, of the tuberculosis inducing germ can generally be selected from the group of proteins which allow a direct and unambiguous correlation to the above-mentioned (poly-)peptides and/or proteins, respectively, especially WhiB2 *Mycobacterium tuberculosis.* Thus, according to the present invention, it is also possible that the (poly-)peptides and/or protein associated with or related to the reproduction, especially cell division, of the tuberculosis inducing germ can be comprised of directly comparable (poly-)peptides and/or proteins, e.g. from the species *Mycobacterium smegmatis,* having the same function and/or at least a comparable structure if compared to the above-mentioned (poly-)peptides and/or proteins, respectively, preferably WhiB2 of *Mycobacterium tuberculosis.*

In this context, taking into consideration the above given definitions, the (poly-)peptide and/or protein associated with or related to the reproduction, especially cell division, of the tuberculosis germ may be a WhmD-like protein, especially a protein of the WhmD-family, preferably WhmD, more preferably WhmD of a species of the genus *Mycobacterium,* even more preferably WhmD of *Mycobacterium smegmatis,* serving as a model system with respect to the inventive concept of the treatment of tuberculosis.

With respect to the WhmD-protein of *Mycobacterium smegmatis,* reference is also made to Fig. 1 wherein the amino acid sequence of this protein is given on the basis of the common one letter code (cf. "WhmDMs" of Fig. 1).

For, it is clearly proved by prior art that both proteins, i.e. WhiB2 of *Mycobacterium tuberculosis,* on the one hand, and WhmD of *Mycobacterium smegmatis,* on the other hand, play an essential role for septum formation and fragmentation, thus exhibiting a comparable physiological function with regard to the respective *Mycobacterium* species. The equivalence in function of WhiB2 of *Mycobacterium tuberculosis,* on the one hand, and WhmD of *Mycobacterium smegmatis,* on the other hand, both being proteins which are associated with or related to the reproduction, especially cell division, of the respective tuberculosis inducing germs, is clearly proven in the prior art (Raghunand and Bishai, 2006a), thus allowing for a direct correlation of these to proteins. This aspect underlying the present invention is especially of high importance with respect to the use of *Mycobacterium smegmatis* and the respective WhmD-protein in view of the finding, identification and modification of pharmacologically active agents being able to inhibit or at least to reduce the expression and/or activity of the (poly-)peptide and/or protein. In other words, results achieved by using the model system of *Mycobacterium smegmatis,* especially with respect to the WhmD-protein as the drug target, can be directly transferred and correlated to pathogenic Mycobacteria germs, like *Mycobacterium tuberculosis.*

As to the active agent exhibiting a pharmacological effectiveness with respect to the tuberculosis inducing germ, according to a preferred embodiment of the present invention, this agent may be in general selected from the group of proteins and (poly-)peptides. Furthermore, the active agent may be selected from the group of proteins and/or (poly-)peptides comprising at least one iron/sulfur-complex and/or having iron/sulfur-coordinating properties. Especially, the active agent may be selected from the group of proteins and/or (poly-)peptides comprising at least one DNA-binding motif, especially at least one helix-turn-helix motif, synonymously also called HTH-motif. Especially, the active agent may exhibit a DNA-binding motif being specific with respect to the gene or DNA-sequence encoding for the protein and/or (poly-)peptide of the tuberculosis inducing germ, said (poly-)peptide and/or protein being associated with or related to the reproduction, especially self division, of the tuberculosis inducing germ. Thus, according to a preferred embodiment of the present invention, the pharmaceutically active agent comprises at least one DNA-binding motif being specific for the gene encoding for WhmD and/or WhiB2, especially as defined above, thereby reducing the gene activity of the respective gene, i.e. having inhibiting and/or suppressing properties.

In this context, the pharmaceutically active agent may comprise at least one iron/sulfur-complex and/or iron/sulfur-complex motif. The (poly-)peptide serving as the active agent preferably may comprise iron/sulfur-coordinating amino acids and/or amino acid residues within the amino acid sequence of the protein, preferably at least one cysteine and/or at least one cysteine residue. In this context, the active agent having an iron/sulfur-complex should comprise a plurality of cysteine amino acids being specifically allocated on the amino acid sequence in order to form the aforementioned iron/sulfur-complex. According to a preferred embodiment of the present invention, the active agent should exhibit at least three cysteine amino acids, preferably four cysteine amino acids, which are preferably allocated and linked with other amino acids in order to form the iron/sulfur complex. In this context, the respective cysteine amino acids should be specifically allocated and distributed on the amino acid sequence of the active agent with the proviso that, especially with respect to the secondary and tertiary structure of the (poly-)peptide, the respective iron/sulfur-complex is formed. In this context, it is clear to the skilled practitioner that also the respective amino acids being allocated between the cysteine residues within the amino acid sequence of the protein also play an important role with respect to the forming of the iron/sulfur-complex. It is preferred that the cysteine residues are spaced in relation to each other within the amino acid sequence in the range of from 1 to 50 amino acid residues, especially 2 to 40 amino acid residues, preferably 3 to 25 amino acid residues. The iron/sulfur-cluster is preferably located in the centre of amino acid sequence and/or directed to the N-terminal end, especially in relation to the HTH-motif. Preferably, the active agent comprises an amino acid sequence on the basis of the sequence from position 73 to 105 according to WhiBTM4 of Fig. 1 or from position 13 to 45 of sequence protocol I.

Furthermore, with respect to the HTH-motif of the active agent, it is preferred that the so-called β-term of the C-terminal HTH-motif comprises 1 to 20 amino acid residues, especially 2 to 15 amino acid residues, preferably 3 to 10 amino acid residues. Especially, the β-term of the HTH-motif of the active agent may comprise the amino acid sequence, according to the one letter code, "GIWGG" or "GVWGG" or functional identical amino acid sequences. In general, the HTH-motif should have a specificity with respect to the WhiB2/WhmD-encoding gene. Preferably, the HTH-motif is located on the C-terminal part of the active agent.

For, applicant has found that proteins having preferably both the Fe/S-cluster and the HTH-motif having a downregulating effect with respect to WhiB2/WhmD.

In addition, the present invention also refers to active agents having at least in part identical or similar motifs or motifs having the same function.

Furthermore, according to the present invention, the active agent may be a viral protein and/or peptide, especially a dsDNA-tailed phage protein. According to a preferred embodiment of the present invention, the active agent may be a bacteriophage protein and/or peptide, more preferably a mycobacteriophage protein and/or peptide, even more preferably a protein and/or peptide of the bacteriophage TM4.

According to a preferred embodiment of the present invention, the active agent may be selected from the group of WhiB-like proteins, especially WhiBTM4 of bacteriophage TM4, and their equally acting derivatives and/or mutations or combinations thereof. In this context, the active agent may also base on WhiBTM4 protein insofar as the active agent comprises at least 40 %, especially at least 60 %, preferably at least 80 % homologous sections and/or regions in the amino acid sequence if compared to WhiBTM4, especially with the proviso that the active agent at least comprises a Fe/S-cluster motif and/or a HTH-motif, preferably a Fe/S-Cluster and a HTH-motif, the Fe/S-cluster motif and/or the HTH-motif being at least functionally identical or similar to the respective Fe/S-cluster motif and/or HTH-motif of WhiBTM4.

For, applicant has surprisingly found out that WhiBTM4 of bacteriophage TM4 specifically leads to a downregulation of WhmD of *Mycobacterium smegmatis* and thus in direct correlation thereto as well to a downregulation of WhiB2 of *Mycobacterium tuberculosis,* thus acting as an active agent with respect to the treatment of tuberculosis and/or serving as the starting point for the identification and/or modification of further pharmacologically active agents having an effectiveness in a comparable manner with respect to the treatment of tuberculosis.

Especially, WhiBTM4 of bacteriophage TM4 exhibits an amino acid sequence as given in Fig. 1 (cf. "WhiBTM4" of Fig. 1). Furthermore, WhiBTM4 of bacteriophage TM4 may be defined by the amino acid sequence as given on the basis of sequence protocol I and/or SEQUENCE LISTING. With respect to the sequence protocol I, the (poly-)peptide and/or protein WhiBTM4 bases on the transcript-ID (locus) NP_569784.

Without being bound to any specific theory, the presence of the aforenamed Fe/S-cluster and/or HTH-motifs leads to the respective properties of the protein comprising these motifs as a repressor/downregulating agent of the gene activity of the gene encoding for WhiB2/WhmD.

With respect to the aforementioned (poly-)peptides and/or proteins "WhiB2", "WhmD" and "WhiBTM4", it is pointed out that also functional analogons can be subsumed thereunder, especially having comparable action profiles and/or having a similar amino acid sequence, e.g. (poly-)peptides being mutated and having for example at least single amino acids exchanged. This, however, is clear to the skilled practitioner.

Moreover, the present invention is not limited to the specific use of the above-mentioned active agents. In general, the active agent can represent any substance being able to inhibit or at least to reduce the expression and/or activity of the (poly-)peptide and/or the protein of the tuberculosis inducing germ, wherein this (poly-)peptide and/or protein is associated with or related to the reproduction, especially cell division, of the tuberculosis inducing germ. Especially, the active agent used within the inventive concept may represent any substance being able to inhibit or at least to reduce the expression and/or activity of the aforementioned WhmD-protein, especially WhmD of *Mycobacterium smegmatis,* and/or WhiB2, especially WhiB2 of *Mycobacterium tuberculosis.*

For example, the active agent may be in general a repressor and/or inhibitor downregulating and/or suppressing the activity of the gene and/or of the DNA-sequence encoding for the above-defined (poly-)peptide and/or protein being associated with or related to the reproduction, especially cell division, of the tuberculosis inducing germ, especially WhiB2/WhmD. In this context, the active agent may be an inhibiting substance.

In general, according to the present invention, it is also possible that the active agent used within the present invention interacts with the gene encoding for the (poly-)peptide and/or protein being associated with or related to the reproduction, especially cell division, of the tuberculosis inducing germ, and/or with the corresponding RNA-sequence and/or with the corresponding protein as such. In this context, the active agent may downregulate or inhibit the activity of the gene or its corresponding RNA or its corresponding protein and/or (poly-)peptide, i.e. the gene product encoded by the DNA-sequence and RNA-sequence, respectively.

According to a further alternative embodiment of the present invention, the active agent may be selected from the group consisting of substances interacting with the enhancer and/or promotor of the gene encoding for the (poly-)peptide and/or protein being associated with or related to the reproduction, especially cell division, of the tuberculosis inducing germ with the proviso that the binding of transcription factors of the tuberculosis inducing germ to the promoter and/or enhancer is inhibited or at least diminished, resulting in a decreased expression of the respective (poly-)peptide and/or protein.

According to another embodiment of the present invention, the active agent may also be selected from substances interacting with a transcription factor as such of the tuberculosis inducing germ, especially with an activator, for the gene or DNA-sequence encoding for the (poly-)peptide and/or protein being associated with or related to the reproduction, especially cell division, of the tuberculosis inducing germ with the proviso that the binding of the mycobacterial transcription factor, especially activator, to the promoter and/or enhancer of the gene encoding for the (poly-)peptide and/or protein being associated with or related to the reproduction, especially cell division, of the tuberculosis inducing germ is inhibited or at least diminished.

In general, according to the inventive concept, the pharmaceutically active agent may be chosen, on the one hand, from the group of substances interacting with the gene and/or DNA- and/or RNA-sequence encoding for the (poly-)peptide and/or protein of the tuberculosis inducing germ as defined above and thus directly or indirectly influencing biosynthesis of the (poly-)peptide and/or protein, e.g. on the transcription- and/or translation-level. On the other hand, the present invention also encompasses embodiments according to which the active agent is chosen from substances directly influencing and/or interacting with the (poly-)peptide and/or protein of the tuberculosis inducing germ as defined above, e.g. in the sense of a competitive or allosteric inactivation and/or blocking of the (poly-)peptide and/or protein of the tuberculosis inducing germ as defined above.

To sum up, the term "inhibitor" and/or "repressor" as used within the present invention preferably refers to a substance diminishing and/or inhibiting and/or prohibiting the gene activity of gene encoding for the (poly-)peptide and/or protein associated with or related to the reproduction, especially cell division, of the tuberculosis inducing germ, especially WhiB2, preferably WhiB2 of *Mycobacterium tuberculosis,* and/or especially WhmD, preferably WhmD of *Mycobacterium smegmatis.* In other words, the inhibitor and repressor, respectively, represents a substance inhibiting and/or preventing or at least diminishing the gene activity on the level of the gene as such, i.e. via direct interaction with the gene or with the promotors and/or enhancers allocated on or associated with the gene, as well as on the level of the gene products, like transcription products (e.g. mRNA) and/or translation products (e.g. proteins, like WhiB2 and/or WhmD). In addition, the pharmacological action of the inhibitor and repressor, respectively, representing the pharmaceutically active agent may affect gene regulating mediators and factors, respectively. In this context, the inhibitor and repressor, respectively, used within the present invention and thus the pharmacologically active agent preferably leads to a diminishment of the expression and gene activity, respectively, for example by direct or indirect interaction with the DNA-sequence or the corresponding RNA-sequence, especially mRNA-sequence, relating thereto. The inhibitor and/or repressor may also interact with the gene product as such, especially in form of the (poly-)peptide and/or protein, preferably WhiB2 and/or WhmD, being encoded by the gene and/or the DNA-sequence. Especially, the inhibitor and/or repressor, respectively, used as the pharmaceutically active agent represents a substance which inhibits or at least reduces the expression and/or activity of the (poly-)peptide and/or protein of the tuberculosis inducing germ, especially of the genus *Mycobacterium,* wherein the (poly-)peptide and protein is associated with or related to the reproduction, especially cell division, of the tuberculosis inducing germ, preferably WhiB2 and/or WhmD.

In this context, the present invention is not limited to specific classes of substances with respect to the active agent. In fact, the active agent can be chosen from the groups of proteins, (poly-)peptides, antibodies, prodrugs, peptidomimetica, small molecules, organic and inorganic low molecular substances, however, with the proviso, that the active agent suppresses, inhibits, inactivates and/or at least diminishes the activity of the (poly-)peptide and/or protein of the tuberculosis inducing germ, said (poly-)peptide and/or protein being associated with or related to the reproduction, especially cell division, of the tuberculosis inducing germ, preferably WhiB2/WhmD.

According to the present invention, the active agent may also be chosen from siRNA (*small interfering RNA*), especially having a high specificity with respect to mRNA encoding for the above-defined (poly-)peptide and/or protein of the tuberculosis inducing germ, especially WhiB2 and/or WhmD.

According to the present invention, the active agent may be administered in an effective amount, especially a pharmacologically effective amount. Furthermore, according to the present invention, the active agent may be systematically administered. In general, the administration of the active agent should be performed in a pharmaceutical composition further comprising at least one pharmaceutically acceptable excipient. However, according to the present invention, the active agent may also be locally and/or topically administered, e.g. in form of an inhalant, especially for inhalation into the lungs. It is also within the scope of the present invention that the administration of the active agent may be performed within the scope of a viral therapy, e.g. via transfection by phages exhibiting a high specificity with respect to the tuberculosis inducing germ, especially *Mycobacterium.* In this context, according to the present invention, also a therapy via transfection using the bacteriophage TM4 may be considered. In general, the therapeutical approach may be performed on the level of incorporating of the gene or DNA-sequence encoding for the active agent into the host (e.g. via transfection using specific phages (carriers) and/or plasmids), i.e. into the tuberculosis inducing germ, especially from the genus *Mycobacterium.*

With respect to the administration of the active agent, it is also within the scope of the present invention that the active agent is administered in form of precursors and/or prodrugs being further metabolized after administration, e.g. within the human body and/or by the host, i.e. the tuberculosis inducing germ as such, into the pharmaceutically active agent.

The respective measurements are known per se to the skilled practitioner so that further explanation referring to these measurements are not required.

Thus, on the whole, the present invention focuses on a purposeful suppression of the gene expression or gene activity of genes encoding for the (poly-)peptide and/or protein of an tuberculosis inducing germ, wherein the (poly-)peptide and/or protein is associated with or related to the reproduction, especially cell division, of the tuberculosis inducing germ. The present invention specifically refers to a gene-knockdown or gene-silencing of WhiB2 encoding genes or of WhmD encoding genes.

To sum up, a new approach for the treatment of tuberculosis is provided on the basis of the inventive use of a pharmaceutical active agent having an effectiveness with respect to a (poly-)peptide and/or protein of a tuberculosis inducing germ, especially of the genus *Mycobacterium,* wherein the (poly-)peptide and/or protein is associated with or related to the reproduction, especially cell division, of the tuberculosis inducing germ, especially WhiB2, preferably WhiB2 of *Mycobacterium tuberculosis,* and WhmD, preferably WhmD of *Mycobacterium smegmatis.*

To sum up, one central gist or core item of the present invention is to be seen in fact that WhiBTM4 has the potential to be able to induce a downregulation and/or a knockout of the phenotype in different species of apathogenic and pathogenic mycobacteria. Such potential results from the fact that WhiB proteins are a common physiological factor of many different mycobacteria, thus representing a universal target for all these species and that for many of them the essential character could be shown by applicant.

According to a **second** aspect of the present invention, the present invention refers to the use of at least one active agent, especially at least one inhibitor and/or repressor, for producing a medicament or pharmaceutical for the prophylactic and/or therapeutic (i.e. curative) treatment of tuberculosis, especially pulmonary tuberculosis, wherein the active agent suppresses or at least downregulates the activity and/or expression of a gene encoding for a (poly-)peptide and/or protein of a tuberculosis inducing germ, especially of a species of the genus *Mycobacterium,* the (poly-)peptide and/or protein being associated with or related to the reproduction, especially cell division, of the tuberculosis inducing germ.

According to this aspect of the present invention, the gene being affected by the pharmacologically active agent preferably represents a gene encoding for the aforementioned WhiB2-protein, especially for the WhiB2-protein of *Mycobacterium tuberculosis*, or especially for the WhmD-protein, especially WhmD of *Mycobacterium smegmatis,* preferable as each defined above.

The term "gene" as used within the present invention also refers to the corresponding nucleic acid molecule or DNA-sequence related thereto. The present invention also refers, as already delineated above, to the RNA-sequence, especially mRNA-sequence, corresponding to the gene encoding for the above-mentioned (poly-)peptide and/or protein, wherein the RNA-sequence represents the posttranscriptional product and wherein the RNA-sequence is complementarily constructed to the codogenic strand of the DNA of the gene. Additionally, the term "DNA-sequence" or "RNA-sequence" also refers to sections of the gene and RNA-fragments and RNA-sections, respectively.

With respect to further details relating to the inventive use of the active agent, reference is made to the explanations with respect to the other aspects, especially with respect to the first aspect, of the present invention, which apply accordingly also to the second aspect of the present invention.

Furthermore, according to a **third** aspect of the present invention, the present invention also refers to the use of a gene encoding for a (poly-)peptide and/or protein of a tuberculosis inducing germ, especially of a species of the genus *Mycobacterium,* and/or of the corresponding DNA-sequence of the gene and/or of the corresponding RNA-sequence and/or of the encoded (poly-)peptide and/or protein as such, the (poly-)peptide and/or protein being associated with or related to the reproduction, especially cell division, of the tuberculosis inducing germ, for finding and/or providing a pharmaceutically active agent and/or substance especially for the prophylactic and/or therapeutic (curative) treatment of tuberculosis, especially pulmonary tuberculosis, wherein the pharmaceutical active agent inhibits or at least reduces the expression and/or activity of the (poly-)peptide and/or the protein of the tuberculosis inducing germ.

As delineated above, the respective (poly-)peptide and/or protein being associated with or related to the reproduction, especially cell division, of the tuberculosis inducing germ is especially represented by the WhiB2-protein, preferably the WhiB2-protein of *Mycobacterium tuberculosis,* or by the WhmD-protein, preferably WhmD of *Mycobacterium smegmatis,* especially as defined above.

In this context, the respective gene especially refers to the aforementioned WhiB2- or WhmD-protein, i.e. specifically encodes for the WhiB2-protein, especially for the WhiB2-protein of *Mycobacterium tuberculosis,* or for the WhmD-protein, especially for the WhmD of *Mycobacterium smegmatis.*

In this context, the aforementioned biomolecules (i.e. the respective gene encoding for the protein, the corresponding DNA-sequence or RNA-sequence and the (poly-)peptide and/or protein as the gene product) preferably represent the drug target for the respective active agent and/or substance, wherein the aforementioned biomolecules can be used for example and in a non-limiting manner within so-called high throughput methods for the screening of possible active agents and/or substances in the sense of the present invention, wherein e.g. substances interacting with the aforementioned molecules can be selected and further investigated with respect to an improved and/or enhanced capability of inhibiting or at least reducing the expression and/or activity of the respective (poly-)peptide and/or protein of the tuberculosis inducing germ, i.e. especially ofWhiB2/WhmD.

The respective screening methods can be performed in-vitro e.g. with respect to the isolated and/or purified biomolecules. Furthermore, it is also possible to perform the respective screening methods in vivo, e.g. within a host as such, like the aforementioned species of the tuberculosis inducing germ, especially of the genus *Mycobacterium.* In this context, preferably the germ *Mycobacterium smegmatis* may be used.

With respect to this aspect of the present invention, the above explanations with respect to the inventive uses according to the first and second aspect of the present invention as well as the explanations as delineated hereinafter also apply accordingly.

According to the present invention, there is also provided - according to a **fourth** aspect of the present invention - a method of identifying and/or retrieving a pharmacologically active agent being able to inhibit or at least to reduce the expression and/or activity of a (poly-)peptide and/or a protein of a tuberculosis inducing germ, wherein the peptide and/or protein is associated with or related to the reproduction, especially cell division, of the tuberculosis inducing germ, the method comprising the steps of
(a) contacting at least one testing molecule with the (poly-)peptide and/or protein being associated with or related to the reproduction of the tuberculosis inducing germ;
(b) detection and/or analysis whether the testing molecule is able to inhibit or at least to reduce the activity of the peptide and/or protein being associated with or related to the reproduction of the tuberculosis inducing germ.

As delineated above, the respective (poly-)peptide and/or protein being associated with or related to the reproduction of the tuberculosis inducing germ is preferably represented by the WhiB2-protein, especially WhiB2 of *Mycobacterium tuberculosis,* or by the WhmD-protein, especially WhmD of *Mycobacterium smegmatis.*

The inventive method can be performed in vitro for example by using isolated (poly-)peptides and/or proteins of the tuberculosis inducing germ and studying the interaction between the testing molecule and the protein, wherein identified testing molecules may be isolated and further investigated with respect to their function of inhibiting or at least reducing the activity of the respective (poly-)peptide and/or protein.

Especially with respect to step (a) of the inventive method, the inventive method can be performed on the basis of a screening method and/or a high throughput (screening) method ("HTS method") being as such well-known to the skilled practitioner and according to which libraries and/or a plurality of testing substances and/or molecules are contacted with the (poly-)peptide and/or protein being associated with or related to the reproduction of the tuberculosis inducing germ and/or according to which testing substances and/or molecules can be selected and further investigated with respect to an improved and/or enhanced capability of inhibiting or at least reducing the expression and/or activity of the respective (poly-)peptide and/or protein of the tuberculosis inducing germ, i.e. especially of WhiB2 and/or WhmD, respectively. In this context, for instance, a microtiter plate may be used comprising a plurality of wells comprising each the preferably isolated (poly-)peptide and/or protein being associated with or related to the reproduction of the tuberculosis inducing germ and the respective testing molecule(s).

Especially, an effective pharmacological target for the testing substance(s) and/or molecule(s) can be represented by a sequence and/or region of the (poly-)peptide and/or protein being associated with or related to the reproduction of the tuberculosis inducing germ, which is of relevance for the physiological function of the protein as such. Especially, the pharmacological target may be represented by the Fe/S-cluster (i.e. iron/sulfur-cluster) motif of the (poly-)peptide and/or protein being associated with or related to the reproduction of the tuberculosis inducing germ, especially the WhiB2-protein, preferably WhiB2 of *Mycobacterium tuberculosis,* and/or especially the WhmD-protein, preferably WhmD of *Mycobacterium smegmatis.* In this context, it has been found that the oxygen-labile Fe/S-cluster motif is of high importance with respect to the physiological functions of the (poly-)peptide and/or protein being associated with or related to the reproduction of the tuberculosis inducing germ. Therefore, the Fe/S-cluster motif represents a highly relevant pharmacological target in the case of the present invention.

Thus, especially according to this aspect of the present invention, the present invention also deals with a method of identifying and/or retrieving a pharmacologically active agent being able to inhibit or at least to reduce the expression and/or activity of a (poly-)peptide and/or a protein of a tuberculosis inducing germ, wherein the peptide and/or protein is associated with or related to the reproduction, especially cell division, of the tuberculosis inducing germ, wherein - especially in step (b) of the inventive method - testing molecules are identified and/or detected which at least partially prevent and/or inhibit the formation of the Fe/S-cluster motif of the (poly-)peptide and/or protein being associated with or related to the reproduction of the tuberculosis inducing germ and/or which at least partially induce the loss and/or denaturation of the Fe/S-cluster motif of the (poly-)peptide and/or protein being associated with or related to the reproduction of the tuberculosis inducing germ.

In a non-limiting manner, the interaction between the testing molecule and the (poly-)peptide and/or protein being associated with or related to the reproduction of the tuberculosis inducing germ can be detected and/or measured optically and/or visually, especially photometrically and/or spectroscopically and/or by other optical and/or visual methods, e.g. by using common photometers. Especially and with respect to the effectiveness of the testing molecule, the aforedescribed prevention and/or inhibition of the formation of the Fe/S-cluster motif of the (poly-)peptide and/or protein being associated with or related to the reproduction of the tuberculosis inducing germ and/or the aforedescibed induction of the loss and/or the denaturation of the Fe/S-cluster motif of the (poly-)peptide and/or protein being associated with or related to the reproduction of the tuberculosis inducing germ can be detected and/or measured optically, especially photometrically. According to one embodiment of the present invention, the influence of the testing molecule on the Fe/S-cluster may be observed photometrically with respect to a sample comprising the testing molecule(s), on the one hand, and the (poly-)peptide and/or protein being associated with or related to the reproduction of the tuberculosis inducing germ, on the other hand, at a wavelength or an OD (optical density) of from 410 to 440 nm, especially from 415 to 430 nm, preferably at about 423 nm, wherein - in otherwise non-denaturating conditions, e.g. under anaerobic conditions - the occurrence of an absorption peak at this wavelength is an indication of the existence of an intact Fe/S-cluster and thus of the ineffectiveness of the testing molecule. In contrast, the disappearing and/or inexistence - in otherwise non-denaturating conditions, e.g. under anaerobic conditions - is an indication that no intact Fe/S-clusters are present and thus an indication for the effectiveness of the testing molecule. In this context, this approach can also be performed starting from denaturated target proteins (e.g. previous treatment under an oxygen-containing atmosphere; no adsorption peak at an OD of 423 nm) and then studying the influence of the testing molecule on the formation and/or reconstitution of the Fe/S-cluster under otherwise non-denaturating and/or reconstitutional conditions, especially under an anaerobic atmosphere (i.e. reconstitution by the addition of specific salts, especially FeCl₃ and an inorganic sulfide such as e.g. Na₂S).

Especially, one approach of the testing of the molecules for their ability to interact with the Fe/S-cluster motif can be performed by observing the reconstitution thereof.

The identified and/or detected testing molecules at least partially preventing and/or inhibiting the formation of the Fe/S-cluster motif and/or at least partially inducing the loss and/or denaturation of the Fe/S-cluster motif may thus exhibit bacteriostatic and/or bacteriocidal and/or antimycobacteriocidal properties with respect to the tuberculosis inducing germ. In order to further prove the bacteriostatic and/or bacteriocidal properties with respect to the tuberculosis inducing germ, the identified and/or detected testing molecules and/or substances are preferably tested for growth inhibition of the tuberculosis inducing germ, preferably in an especially separate testing assay, e.g. in an in-vivo testing assay.

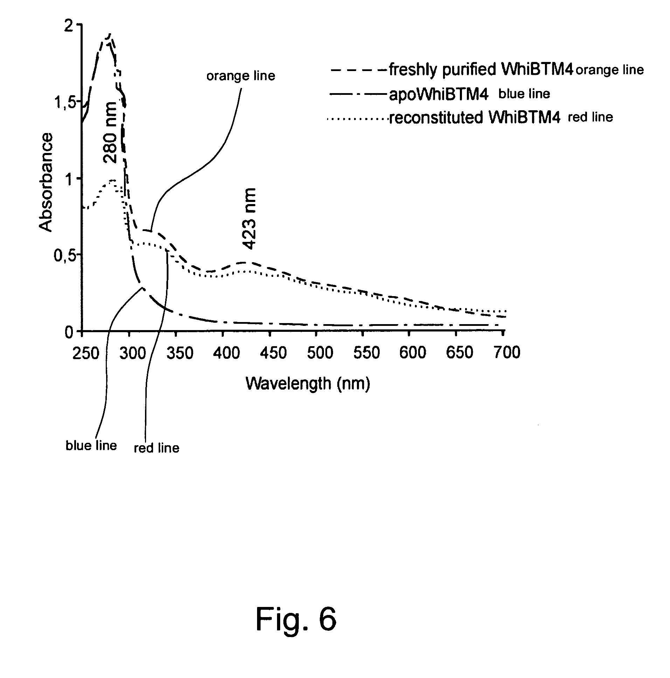

For further details with respect to this aspect of the present invention, reference is made to the following description of Fig. 6, which applies accordingly.

Preferably, the inventive method may also be performed in vivo, using a respective host, e.g. *Mycobacterium segmatis* according to which a direct correlation to the pathogenic tuberculosis inducing germs can be performed. The effectiveness of the testing molecules may be determined for example and in a non-limiting manner by the respective analysis of the targeted (poly-)peptide and/or protein, e.g. by functional and/or structural analysis and the like. The respective methods are well-known to the skilled practitioner thus requiring no further explanations.

However, for further explanations, reference is made to the further aspects of the present invention.

The present invention also refers - according to a **fifth** aspect of the present invention - to a method of identifying and/or retrieving a pharmacologically active agent being able to inhibit or at least to reduce the expression and/or activity of a (poly-)peptide and/or a protein of a tuberculosis inducing germ, wherein the (poly-)peptide and/or protein is associated with or related to the reproduction, especially cell division, of the tuberculosis inducing germ, the method comprising the steps of:
(a) incorporating at least one testing substance and/or a DNA-sequence encoding for the testing substance and/or its respective RNA-sequence into a germ being associated with tuberculosis and being able to express the (poly-)peptide and/or the protein of the tuberculosis inducing germ, wherein the (poly-)peptide and/or protein is associated with or related to the reproduction, especially all division, of the tuberculosis germ, the germ being preferably selected from the group of the genus *Mycobacterium;*
(b) detection and/or analysis whether the testing substance is able to inhibit or at least to reduce the expression and/or activity of the (poly-)peptide and/or protein being associated with or related to the reproduction of the tuberculosis inducing germ.

This inventive method according to the fifth aspect of the present invention thus focuses on an in vivo testing approach according to which a specific host is used. In this context, with respect to the identifying and/or retrieving of pharmacological active agents, the species *Mycobacteria segmatis* is preferred.

In a non-limiting manner, the method can be performed by transfection of the host, e.g. by using the bacteriophage TM4 comprising the respective DNA-sequence encoding for the testing substance, e.g. in form of or incorporated into a specific plasmid. After transfection and expression of the testing substance, i.e. a (poly-)peptide or protein with the influence on the respective host protein, e.g. WhiB2/WhmD, can be investigated. For this, the respective expressions and/or activity studies and/or structural analysis and the like of the protein to be affected may be performed. Especially, the effectiveness of the testing substance is evaluated on the basis of the viability of the respective cells and/or colonies. The inventive method mainly focuses on the influence of testing molecules on WhiB2/WhmD preferably on the level of gene activity. However, the inventive method is not limited thereto and also comprises approaches focusing on an inactivation of WhiB2/WhmD as such, i.e. on the level of the gene product.

One inventive concept with respect to the identification and/or modification of pharmacologically active agents in the sense of the present invention, i.e. substances having an influence on the activity especially of WhmD and/or WhiB2, may be seen in the approach according to which the pharmacologically active agent WhiBTM4, especially as defined above, serves - in the manner of speaking - as the starting point for the identification and/or retrieving of pharmacologically equivalent active agents. In this context, one inventive approach may be performed by realizing specific mutations with respect to the DNA encoding for WhiBTM4 and studying the resulting influence of the mutation with respect to the pharmacological effectiveness of the resulting mutated protein structures, i.e. whether its downregulating effect on WhiB2/WhmD is affected. In this context, pharmacologically relevant motifs and/or protein regions may be identified, especially with respect to their importance in view of the pharmacological effectiveness. On the basis of the identified pharmacological important motifs of the protein, like the above defined iron/sulfur-cluster and/or the above defined helix-turn-helix-motif (HTH-motif), further modifications in form of mutations can be performed, preferably excluding these pharmacologically important motifs, in order to further optimize and/or improve the effectiveness of the protein, for example in order to develop so-called small molecules having the above named motifs and further comprising improved properties e.g. with respect to their capability to penetrate through the rigid wall of the pathogenic germ (improved endocytotic properties).

Thus, to sum up, on the one hand, with respect to the introduction of specific concerned regions of the active agent and/or testing substance, e.g. basing on the WhiBTM4-protein may be identified, and, on the other hand, these conserved regions with respect to the pharmacological efficiency in the sense of the present invention may serve as basic principles for the development of other pharmaceutically active agents with further improved properties, e.g. with respect to a further enhanced pharmaceutical effectiveness, reduced siteeffects, better incorporation into the pathogenic germ, and the like.

The respective methods are known to the skilled practitioner. For further details reference is made to the further annotations given.

However, the aforementioned method is not limited to the identification of (poly-)peptides and/or proteins with respect to their effectiveness with respect to the (poly-)peptide and/or protein of the host to be affected. In fact, all kind of potential active agents may be identified and/or retrieved, including peptidomimetica, small drugs as well as small organic or inorganic molecules.

In general, the detection of the interaction and/or influence of the testing substance with respect to the (poly-)peptide and/or protein to be affected, especially WhmD/WhiB2 may be performed with respective methods being well-known to the skilled practitioner.

In case that the identified and/or modified active agent is in form of a (poly-)peptide and or protein, the present invention also refers to the DNA-sequence and/or RNA-sequence encoding for the identified and/or modified active agent as well as to vectors comprising the respective DNA-sequence and/or RNA-sequence.

With respect to the method according to this aspect of the present invention, the respective explanations given with respect to the remaining aspects of the present invention apply accordingly.

The present invention also refers - according to a **sixth** aspect of the present invention - to a method of improving the pharmacological properties of a pharmacologically active agent identified by one of the above defined methods, the method comprising the steps of
(a) identifying and/or locating the binding site of the pharmacologically active agent to the (poly-)peptide and/or protein being associated with or related to the reproduction of the tuberculosis inducing germ and/or identifying and/or locating the binding site of the pharmacologically active agent to the DNA-Sequence, especially to the gene, and/or to the corresponding RNA-Sequence, encoding for the (poly-)peptide and/or protein being associated with or related to the reproduction of the tuberculosis inducing germ;
(b) modifying the binding site of the pharmacologically active agent, especially via molecular modelling; and
(c) modifying the pharmacologically active agent with the proviso that the specificity and/or affinity and/or avidity of the binding site of pharmacologically active agent is optimized and/or increased with respect to the (poly-)peptide and/or protein being associated with or related to the reproduction of the tuberculosis inducing germ and/or with the proviso that the specificity and/or affinity and/or avidity of the binding site of pharmacologically active agent is optimized and/or increased with respect to the DNA-Sequence, especially to the gene, and/or to the corresponding RNA-Sequence.

In this context, the binding site of the active agent, especially the aforementioned HTH-motif, preferably of WhiB2/WhmD may be further identified by mutagenesis, especially site-directed mutagenesis, which is known per se to the skilled practitioner. The optimized binding site can then form the basis for equivalent active substances as delineated above.

For further explanations, reference is made to the further aspects of the present invention, which apply accordingly.

The present invention also refers - according to a **seventh** aspect of the present invention - to a method of modification of a pharmacologically active agent identified or improved by one of the above defined methods, wherein the pharmacologically active agent is further modified in order to attain
(i) a modified active centre, a modified activity spectrum and/or a modified organ specificity, especially lung specificity, and/or
(ii) improved properties with respect to the incorporation and/or uptake properties with respect to the tuberculosis inducing germ and/or
(iii) an improved activity and/or
(iv) a reduced toxicity (an improved therapeutic index) and/or
(v) reduced side effects and/or
(vi) a reduced toxicity (an improved therapeutic index) and/or
(vii) a retarded onset and/or beginning of the therapeutic activity and/or duration of the therapeutic activity and/or
(viii) modified pharmacological parameters, especially resorption, distribution, cell penetration, metabolism and/or excretion, and/or
(iv) modified physicochemical parameters, especially solubility, hygroscopic properties, colour, flavour, smell, stability, application form, and/or
(x) a modified general specificity, especially organ/tissue-specificity, and/or
(xi) a modified application form and/or application route, especially by
   (a) esterification of carboxyl groups and/or
   (b) esterification of hydroxyl groups with carbonic acids and/or
   (c) esterification of hydroxyl groups, especially to phosphates, pyrophosphates, sulfates and/or hemisuccinates and/or
   (d) formation of pharmaceutical acceptable salts and/or
   (e) formation of pharmaceutical acceptable complexes and/or
   (f) synthesis of pharmacological polymers and/or
   (g) introduction of hydrophilic groups and/or
   (h) introduction and/or exchanging of isosterical and/or bioisosterical groups and/or
   (i) synthesis of homologous compounds and/or
   (j) introduction of branched side chains and/or
   (k) introduction and/or exchange of substituents in aromatic groups and/or side chains and/or modifying of substituent distribution and/or aubstituent arrangement and/or
   (l) conversion of alkyl substituents into cyclic analoga and/or
   (m) derivatization of hydroxyl groups into ketals and/or acetals and/or
   (n) N-acetylization into amides and/or phenylcarbamates and/or
   (o) synthesis of Mannich-bases and/or imines and/or
   (p) conversion of ketones and/or aldehydes into Schiff bases, oximes, acetals, ketals, enolesters, oxazolidines, thiozolidines and combinations thereof.
   (q) reduction of the molecular size, preferably be deletion and/or exchange of amino acids, however, with the proviso that pharmacologically effective motifs of the pharmacologically active agent are at least essentially not affected.

Especially the aforementioned Fe/S-cluster motif and/or the HTH-motif should not be affected by the above modifications. With respect to this method, also the pharmacologically active protein WhiBTM4 as well as all pharmacological active agents as given with respect to the further aspects of the present invention, especially with respect to the first aspect of the present invention, may serve as the starting point for further modification. This applies to the other methods of the invention accordingly.

In general, respect to the inventive method according to this aspect of the present invention, reference can be made to the explanations given with respect to the further aspects of the present invention which apply accordingly.

The present invention also refers - according to an **eighth** aspect of the present invention - to a pharmaceutically active agent, especially for the prophylactic and/or therapeutic (i.e. curative) treatment of tuberculosis, especially pulmonary tuberculosis, the pharmacologically active agent being able to inhibit or at least to reduce the expression and/or activity of a (poly-)peptide and/or a protein of a tuberculosis inducing germ, the (poly-)peptide and/or protein being associated with or related to the reproduction, especially cell division, of the tuberculosis inducing germ, wherein the pharmacologically active agent is obtainable by the inventive methods given above and/or wherein the pharmacologically active agent is defined according to the further aspects of the present invention.

The present invention also refers - according to an **ninth** aspect of the present invention - to a pharmaceutical composition, especially for the prophylactic and/or therapeutic (i.e. curative) treatment of tuberculosis, especially pulmonary tuberculosis, the pharmaceutical composition comprising an efficient amount, especially a pharmacological effective amount, of at least one pharmacologically active agent being able to inhibit or at least to reduce the expression and/or activity of a (poly-)peptide and/or a protein of a tuberculosis inducing germ, the (poly-)peptide and/or protein being associated with or related to the reproduction, especially cell division, of the tuberculosis inducing germ, wherein the pharmacologically active agent is obtainable by the methods as described above and/or wherein a pharmacologically active agent is used as defined above. For example, pharmacologically active agents on the basis of the WhiBTM4-protein may be used. For example, pharmacologically active agents comprising the aforementioned iron/sulfur-clusters and/or the helix-turn-helix-motif may be used.

For further explanations with respect to the pharmaceutical compositions of the present invention, reference is made to the explanations given with respect to the other aspects of the present invention which apply accordingly.

Finally, according to a **tenth** aspect of the present invention, there is provided a method of treating a human suffering from tuberculosis, especially pulmonary tuberculosis, the method comprising: administering an efficient amount of at least one pharmacologically active agent being able to inhibit or at least to reduce the expression and/or activity of a peptide and/or a protein of a tuberculosis inducing germ, the peptide and/or protein being associated with or related to the reproduction, especially cell division, of the tuberculosis inducing germ.

With respect to the inventive method according to this aspect, the explanations given with respect to the further aspects of the present invention apply accordingly.

Moreover, it is clear to the skilled practitioner that one substance, especially one pharmaceutically active agent, being provided by or defined in one specific aspect of the present invention can be used within another aspects of the present invention accordingly.

To sum up, the present invention refers to a method according to which species of the genus *Mycobacterium* are infected by a mycobacterium-specific phage TM4 comprising a gene sequence encoding for a WhiB-like protein of *Streptomyces coelicolor.* The analysis of the effect of the expressed protein on the phenotype of the bacteria and the comparison with effects of mutated analoga result in the identification of active components or motifs in the structure of WhiB-like proteins. These components can then, form the basis for small molecules which can be used in the therapy of tuberculosis and other diseased induced by mycobacteria.

As delineated above, mycobacteria require, for septation and cell *division, inter alia,* proteins which belong to the family of WhiB-like proteins. In this context, applicant has shown in his experimental work that a transfection of *Mycobacterium smegmatis* with a specific expression plasmid, i.e. pSD24:WhiBTM4, encoding for a WhiB-like protein (WhiBTM4) results in a significant cell division defect, i.e. a hindered septation and cell division as well as in a loss of viability. The functional WhiBTM4-protein comprising the Fe/S-cluster seems to compete against wild-type WhiB2/WhmD e.g. in the sense of a selective downregulation of WhiB2/WhmD, resulting in a knockout of the essential function of the native protein of the host. This, however, leads to an abnormal growth of the host bacterium.

Alternatively, *Mycobacterium smegmatis* may also be infected/transfected with mycobacteria phage TM4 as such comprising the information of a WhiB-like protein (WhiBTM4). This infection/transfection may also result in a novel expression of a functional protein which comprises, especially, as a co-factor an iron/sulfur-cluster (Fe/S-cluster). This may also lead to the phenomenon that the mycobacteria surprisingly exhibit a WhiB2/WhmD-gene-knockout-phenotype, resulting in a hindered septation and cell division as well as in a loss of viability.

On the whole, these models and/or approaches allow, especially on the basis of an analysis of mutations of the introduced protein, for identifying active components and/or motifs in the WhiB-like proteins being responsible for the specific competition against and/or downregulation and/or knockout of wild-type WhiB2/WhmD. The identified active components and/or motifs can then serve as starting basis for respective therapeutical active agents or the development thereof. In this context, the active agents may comprise or consist of the protein as such. Furthermore, the active agents may, on the one hand, comprise or consist of the identified active components and/or motifs as such (each component on its own or in their specific combination) or, on the other hand, may alternatively be constructed as specific analogons with respect to the therapeutic effectiveness of the identified components and/or motifs. As delineated above, the active agent can be selected from the groups of proteins, (poly-)peptides, antibodies, prodrugs, peptidomimetica, small molecules, organic and inorganic low molecular substances.

Thus, the present invention also refers to a method for analysis of WhiB-like proteins of mycobacteria, according to which active components and/or motifs of the protein can be identified. In a final stage, small molecules, for example in form of peptides, can be provided, being small enough to penetrate the rigid wall of mycobacteria. This peptide can represent a potential pharmaceutically active agent for the therapy of diseases, like tuberculosis induced by mycobacteria.

It is also within the scope of the present invention to use the aforementioned testing methods and/or approaches on the basis of a transfection with a specific expression plasmid or on the basis of an infection/transfection with a specific phage in order to test for the possible therapeutic effectiveness in the sense of the present invention of other molecules, especially proteins, being expressible in mycobacteria as the respective host.

Further embodiments, modifications and variations of the present invention are obvious to the skilled practitioner by reading the present specification and/or can be implemented by him without leaving the scope of the present invention.

The present invention is illustrated on the basis of the following detailed explanations and exemplary embodiments as well as experimental data, which, however, do not limit the present invention in any way.

In the following, a detailed description of the inventive concept is given on the basis of applicant's studies and investigations, respectively, comprising experimental data which focus on the WhiB2/WhmD polypeptide target of *Mycobacterium* and the specific impact of active agents on the expression and/or activity profile of the aforementioned polypeptides.

### Detailed explanation and examplification of the present invention:

WhiB-like proteins of actinomycetes are iron-sulfur (Fe-S) proteins and are believed to have regulatory functions in many essential bacterial processes. The systematic determination of the genome sequences of mycobacteriophages has revealed the presence of several WhiB-like genes. Here applicant focuses on the WhiB-like protein of mycobacteriophage TM4, WhiBTM4, and reports for the first time that a viral protein is capable of co-ordinating a Fe-S cluster (Fe/S-cluster, iron/sulfur-cluster). The WhiBTM4 UV- visible absorption spectrum shows the presence of an oxygen sensitive [2Fe-2S] cluster. Applicant demonstrates that WhiBTM4 senses normoxia, hypoxia and the presence of reducing or oxidizing agents through its cluster degradation and reconstitution. Over-expression of WhiBTM4 in the mycobacterial host leads to hindered septation and cell division resembling a WhiB2/WhmD knock-out phenotype. The quantification of RNA-levels during phage infection shows that WhiBTM4 is a highly transcribed early phage gene leading to a down-regulation of WhmD. Mutational analysis of WhiBTM4 based on the observed phenotype reveals that the Fe-S complex co-ordinating cysteines and amino acids of the C-terminal helix-turn-helix motif are mandatory for growth inhibition by WhiBTM4. In Conclusion, applicant demonstrates that WhiBTM4 is an early Fe-S protein that by competing with Whib2/WhmD leads to aberrant growth of the host.

Fe-S clusters represent one of the simplest and most functionally versatile prosthetic groups (Beinert *et al*., 1997). By undergoing oxidation-reduction reactions they play important roles in metabolic pathways and regulatory processes across all kingdoms of life. In these clusters Fe ions are linked to each other through sulphide bridges on a cysteine rich protein scaffold. The family of WhiB proteins of actinomycetes are putative transcription factors which could be identified in all actinomycetes sequenced so far, but not in other organisms. The majority of the WhiB-like proteins contain four perfectly conserved cysteines. WhiD, a protein of *Streptomyces coelicolor* which is required for late stages of sporulation, was the first WhiB protein shown to co-ordinate a Fe-S cluster with the help of these essential cysteines (Jakimowicz *et al*., 2005).

The determination and annotation of the *Mycobacterium tuberculosis* genome sequence revealed the presence of seven WhiB-like genes in this organism (WhiB1 to Whib7) (Cole *et al*., 1998; Soliveri *et al.,* 2000). The proteins are characterized by the presence of four invariant cysteine residues and a C-terminal helix-turn-helix (HTH) motif with a GV/IWGG amino acid sequence signature in the putative β-turn. Though these motifs are conserved in all seven WhiB proteins, their cellular functions seem to differ substantially and are believed to involve antibiotic resistance, pathogenesis, cell division and stress response (Alam *et al.,* 2007; Gomez and Bishai, 2000; Morris *et al.,* 2005; Steyn *et al.,* 2002). Fe-S cluster co-ordinating properties have recently been shown for all *M tuberculosis* derived WhiB proteins (Alam *et al.,* 2007, 2008; Singh *et al.,* 2007). Though several reports on mycobacterial WhiB proteins give insight into their possible function as regulatory proteins, the precise molecular mechanism and the signal transduction pathways involved are not yet understood.

The systematic sequencing and comparison of bacteriophage genomes revealed the presence of WhiB-like proteins in several mycobacteriophages and streptomyces phages (Morris *et al.,* 2008; Pedulla *et al.,* 2003; Van Dessel *et al.,* 2005). Mycobacteriophages have proven useful tools for the exploration of mycobacterial genetics. Furthermore, the recent sequencing of more than 30 mycobacteriophage genomes revealed an extraordinary high genetic diversity among these phages (Hatfull *et al*., 2006; Pedulla *et al*., 2003). The variety and high number of phage genes of bacterial origin reflect the frequent recombination events which were probably highly important for both virus and host. However, the interaction of mycobacteriophage and mycobacterium during the phage replication cycle is not yet fully understood and only a few phage proteins have been expressed and examined in detail.

In the present invention, applicant focuses on mycobacteriophage TM4. This dsDNA-tailed phage is fully sequenced and has a broad host range among the fast and slow growing mycobacteria including *M. tuberculosis, Mycobacterium bovis* bacille Calmette-Gudrin (BCG), *Mycobacterium ulcerans* and *M. smegmatis* (Ford *et al*., 1998; Rybniker *et al*., 2006). The annotation of the TM4 genome showed a single putative WhiB-like protein situated in a genomic region which encodes primarily for regulation- and DNA metabolism-associated proteins. This protein was named WhiBTM4. The alignment of the amino acid sequence of WhiBTM4 with the seven mycobacterial WhiB proteins identified so far revealed high homology with 66 amino acids of the C-terminal part of WhiB2 (Fig. 1). The N-terminal 56 amino acids of WhiB2, which are of unknown function, are missing in the short bacteriophage protein. WhmD (WhiB2 of *M. smegmatis*) was shown to be an essential gene for cell division and a conditional WhmD mutant exhibited irreversible filamentous and branched growth with aberrant septum formation (Gomez and Bishai, 2000). This conditional mutant could be complemented by both WhmD of *M. smegmatis* and WhiB2 of *M tuberculosis,* showing that these proteins are functionally equivalent (Raghunand and Bishai, 2006a). Its role in mycobacterial cell division as well as its essential nature and uniqueness to mycobacteria makes WhiB2 an interesting drug target.

Applicant shows that over-expression of WhiBTM4 leads to a WhmD knock-out phenotype in *M smegmatis* most likely by down-regulating WhmD expression. The spectroscopic analysis of the purified protein suggests that WhiBTM4 co-ordinates a [2Fe-2S] cluster which is oxygen sensitive and can be restored under anaerobic conditions. A random PCR based mutagenesis method is used for the identification of WhiBTM4 amino acids which are essential for the growth inhibitory phenotype. 17 amino acids are found to be relevant for proper function of the protein and the mutations revealed that both Fe-S cluster co-ordinating properties as well as amino acid residues of the C-terminal HTH-motif are essential for the observed cytotoxicity. These data support the hypothesis that WhiB proteins are involved in signal transduction using the conserved C-terminal region which is also present in WhiBTM4 (Soliveri *et al*., 2000). Interestingly, *M smegmatis* colonies expressing WhiBTM4 proteins with mutations other then the cysteine at position 39 show a red to orange colony morphology, most likely indicating the non-toxic accumulation of the Fe-S complex, implicating an additional and so far undescribed role for WhiB-like proteins as iron-storage proteins

### Overexpression of WhiBTM4 in M. smegmatis leads to a WhmD knock-out phenotype

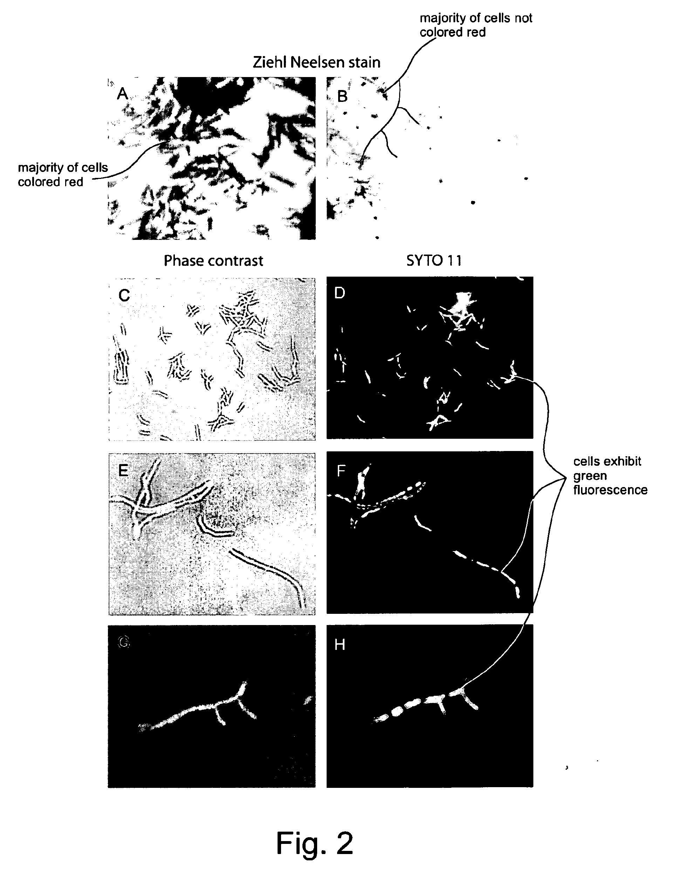

It was recently shown that WhmD-depletion in a conditionally complemented *M smegmatis* WhmD knock-out mutant led to defects in cell division thus establishing WhmD as an essential mycobacterial protein (Gomez and Bishai, 2000). Cells lacking WhmD formed aggregates and the microscopic examination showed long, branched and filamented cells. Over-expression of WhiBTM4 in *M smegmatis* from the acetamidase inducible promoter leads to a similar phenotype (Fig. 2). After 12 h of induction with 0.02 % acetamide in liquid media, cells are elongated, highly branched (Fig. 2 E, G) and have lost their acid-fast properties in the Ziehl Neelsen stain (Fig. 2 B). Fluorescence microscopy with SYTO11 which specifically stains nucleic acids reveals the presence of multiple nucleoids in the filamented cells (Fig. 2 F, H). Thus, over-expression of WhiBTM4 in liquid medium leads to a phenotype similar to the WhmD knock-out mutant with hindered septum formation and fragmentation. Expression of WhiBTM4 on agar plates containing acetamide is not tolerated by *M smegmatis* (not shown).

### WhiBTM4 is highly expressed during the early phase of phage replication and leads to a WhmD down-regulation.

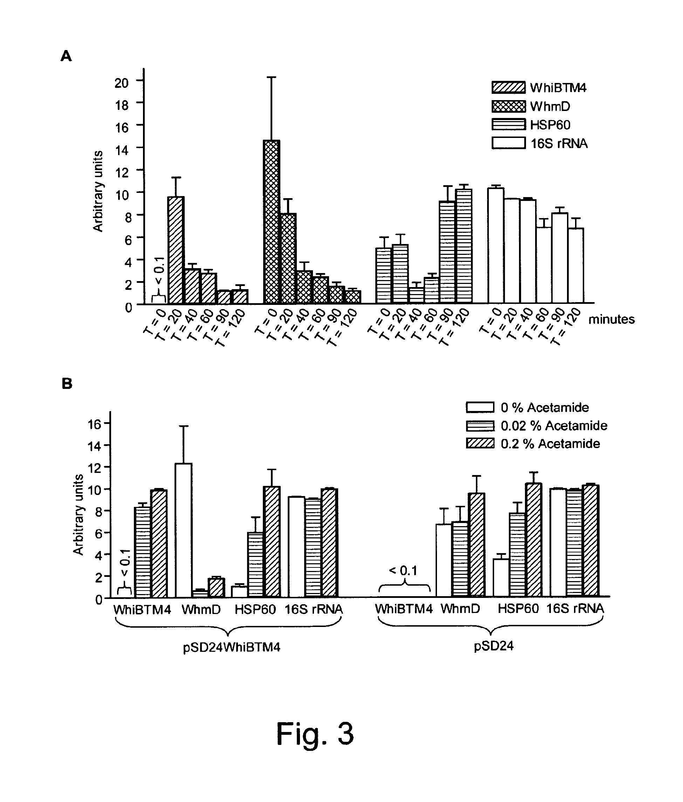

Since it is not clear whether and when phage derived WhiB-like proteins are expressed during the replication cycle, applicant was interested in the expression pattern of WhiBTM4 during phage infection. *M smegmatis* cells are infected with WhiBTM4 and mycobacterial RNA is extracted at different time-points. mRNA levels of WhiBTM4 and three host genes (WhmD, HSP60 and 16S rRNA) are determined using qRT-PCR. This experiment reveals that the phage gene is highly transcribed 20 minutes after phage infection and expression levels gradually decline at later time-points (Fig. 3A). This observation establishes WhiBTM4 as an early phage gene. WhmD expression levels decrease in the presence of WhiBTM4 whereas 16S rRNA levels are unchanged until the late phase of infection. HSP60 is slightly down-regulated after 40 minutes of infection but levels increase during the late phase of phage replication. These findings are supported by similar data in *M smegmatis* cells harbouring pSD24WhiBTM4. Here, induction of WhiBTM4 expression with acetamide for four hours leads to a 90% decrease in WhmD-specific RNA (Fig. 3B). The actual copy numbers determined in the experiments are given in table S1.

### Purification of WhiBTM4

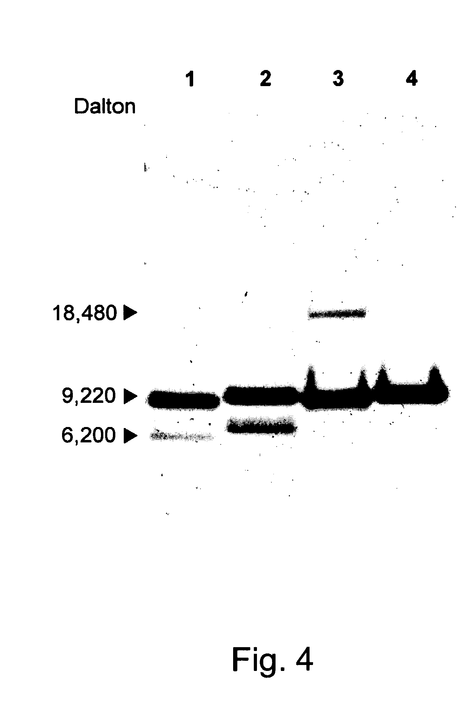

Over-expression of 6x His-tagged WhiBTM4 in *Escherichia coli* BL21 with subsequent loading of cell lysate to a Ni-NTA column leads to a deep brown coloring of the column. When purified and eluted under native conditions, total yield of protein is low and most of the brown substance remains on the column, indicating the formation of protein aggregates. However, the soluble fraction has a light brown colour and the eluates can be further examined by SDS-PAGE and UV-visible absorption spectroscopy. Purification and elution in the presence of 8 M urea leads to a rapid clearance of the column and elution of a dark brown fluid. On SDS-PAGE the eluates of both natively purified protein and protein isolated in the presence of 8 M urea show the presence of highly pure WhiBTM4 with prominent bands at approximately 9 kDa (Fig. 4). Protein samples loaded without reducing agents such as dithiothreitol (DTT) reveal a second band at 18 kDa which disappears in the presence of 1mM DTT. All visible bands are examined by peptide mass fingerprint (PMF). The peptide pattern was consistent with His-tagged WhiBTM4. The second larger band at 18 kDa which resolves in the presence of reducing agents shows that WhiBTM4 is capable of forming protein dimers. Furthermore, in the presence of DTT the prominent bands at 9 kDa migrate slower through the gel, most likely through the reduction of intramolecular disulfide bonds formed between the four cysteine residues of the protein leading to a less compact protein formation (Fig. 4). This phenomenon has also been observed in other WhiB proteins (Raghunand and Bishai, 2006b).

Samples loaded onto SDS-gels are also examined by MALDI-TOF for an accurate analysis of protein mass (Fig. 4). Here, the major peak is detected at 9220 Dalton which corresponds to the calculated protein mass of the 6xHIS-tagged WhiBTM4.

### Evidence for WhiBTM4 co-ordinating an oxygen sensitive [2Fe- 2S] cluster

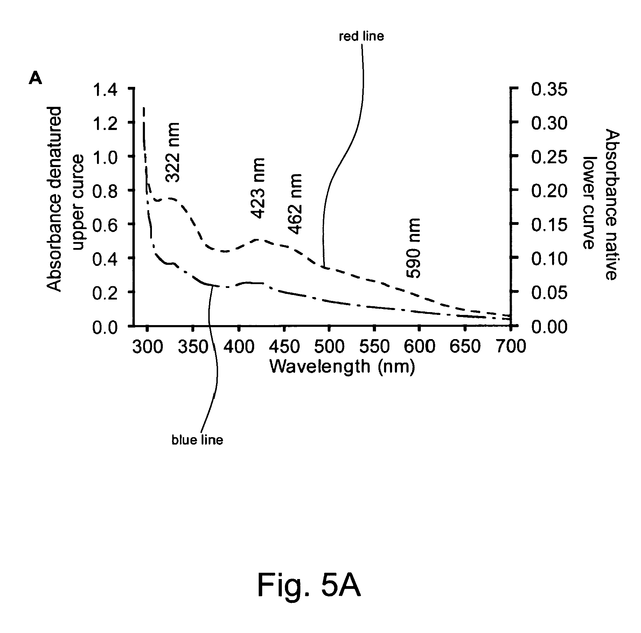

The brown appearance of purified WhiBTM4 suggests the presence of an iron-sulfur chromophore. Samples are examined for their UV-visible absorption spectra which showed peaks at 322 and 423 nm (Fig. 5A). The highly concentrated cluster extracted with buffers containing 8 M urea shows two additional shoulders at 462 and 590 nm. This pattern is characteristic for mycobacterial proteins carrying a [2Fe-2S] cluster as well as for [2Fe-2S] ferredoxins from other organisms (Alam *et al.,* 2007; Messerschmidt, 2001). The A₄₂₃/A₂₈₀ ratio of freshly purified WhiBTM4 is 0.25. With 0.34 atoms of iron per WhiBTM4 monomer the amount of protein containing the cluster was substoichiometric (Table 1). Applicant notes that the presence of 8 M urea has no effect on cluster stability indicating that the WhiBTM4 cluster is resistant to chaotropic agents.

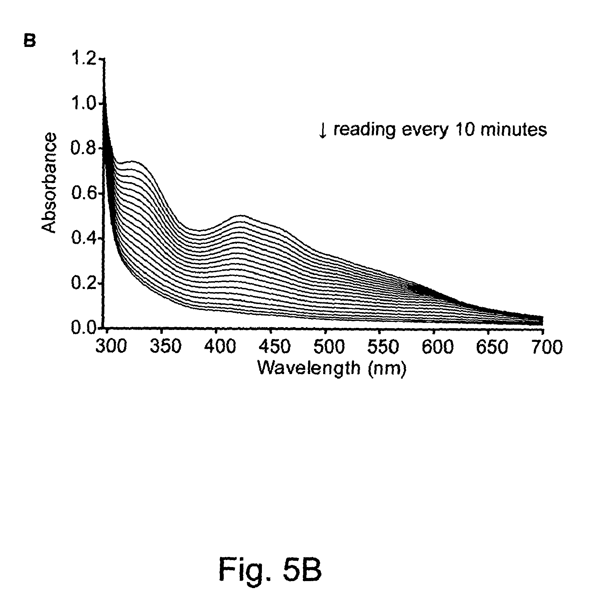

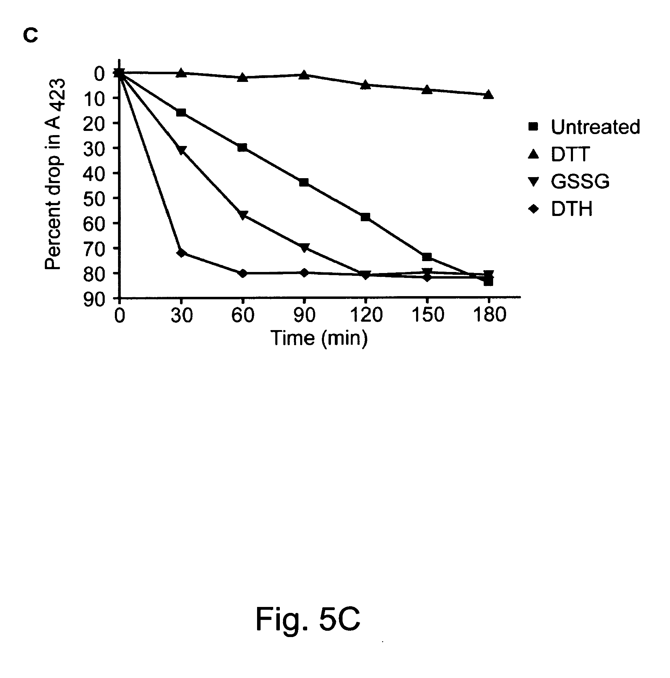

Iron-sulfur clusters are known to sense the presence of various oxidants and reductants making them intracellular redox sensing molecules. Applicant was interested in similar properties of the mycobacteriophage TM4 derived Fe-S cluster. Applicant demonstrates that the intensity of the characteristic UV-visible absorption peaks decreases when the protein is exposed to atmospheric oxygen. The peaks as well as the intense brown colour of the protein are lost after three hours indicating that the cluster is oxygen sensitive (Fig. 5B). This rapid cluster degradation observed in WhiBTM4 stands in contrast to WhiB proteins of the host where cluster loss through atmospheric oxygen is achieved within days (Alam *et al*., 2008). In WhiBTM4 cluster disassembly can be arrested in the presence of 10mM DTT which protects Fe-S clusters as an antioxidant and inhibits the formation of intramolecular disulphide bonds (Fig. 5C). On the contrary the decay of the WhiBTM4 Fe-S cluster can be enhanced by the addition of 10 mM oxidized glutathione (GSSG) indicated by a faster drop of the peak at A₄₂₃ (Fig. 5C). The characteristic light absorptions of [2Fe-2S] clusters are generated by sulfur-Fe³⁺ charge transfer transitions

(Messerschmidt, 2001). Thus, a brown colored [2Fe-2S]²⁺ cluster can be reduced with the reducing agent dithionite to a [2Fe-2S]⁺ cluster which results in visible bleaching of protein and a characteristic change in the UV-visible absorption spectrum (rapid loss of the peak at A₄₂₃ and appearance of a peak at A₃₂₂). This UV-visible change of charge rather than disassembly of cluster is also observed in WhiBTM4 (Fig. 5C).

Recently it was shown that fully oxygenated WhiB4 (apoWhiB4) of *M. tuberculosis* is capable of co-ordinating an [4Fe-4S] cluster under semi-anaerobic conditions in the presence of DTT (Alam *et al*., 2007). During anaerobic incubation of colorless apoWhiBTM4 with FeCl₃, Na₂S and DTT, a characteristic brown colour developed within three to four hours. The peak pattern of the UV-visible absorption spectra of this reconstituted WhiBTM4 was similar to that of freshly purified protein indicating that apo WhiBTM4 can be reconstituted to a [2Fe-2S] cluster (Fig. 6). During anaerobic incubation and dialysis a substantial amount of protein had aggregated. However, the A₄₂₃/ _{A28}0 ratio of reconstituted WhiBTM4 is higher than the ratio of the freshly purified cluster and with 0.39 this ratio indicates the presence of an intact [2Fe-2S] cluster (Rouhier *et al*., 2007). This is mirrored by the high number of iron atoms per monomer/dimer of WhiBTM4 which is 1.13 and 2.26 respectively as determined by atom absorption spectroscopy (Table 1).

Despite several trials with different reducing agents such as dithionite and sodium aspartate, applicant was not able to generate an electroparamagnetic resonance (EPR) signal from either freshly purified or reconstituted protein. EPR negativity in WhiB proteins seems to be a common problem (Alam *et al*., 2008). However, it is consensus that freshly purified WhiB proteins with UV-visible absorption spectra similar to the ones applicant obtained carry a [2Fe-2S] cluster (Alam *et al*., 2007, 2008; Jakimowicz *et al*., 2005). This assumption is supported by identical UV-visible absorption spectra from well defined [2Fe-2S] cluster co-ordinating proteins such as the ferredoxins (Messerschmidt, 2001).

### Both the conserved cysteines and the C-terminal HTH-motif are involved in the WhiBTM4 growth inhibitory effect

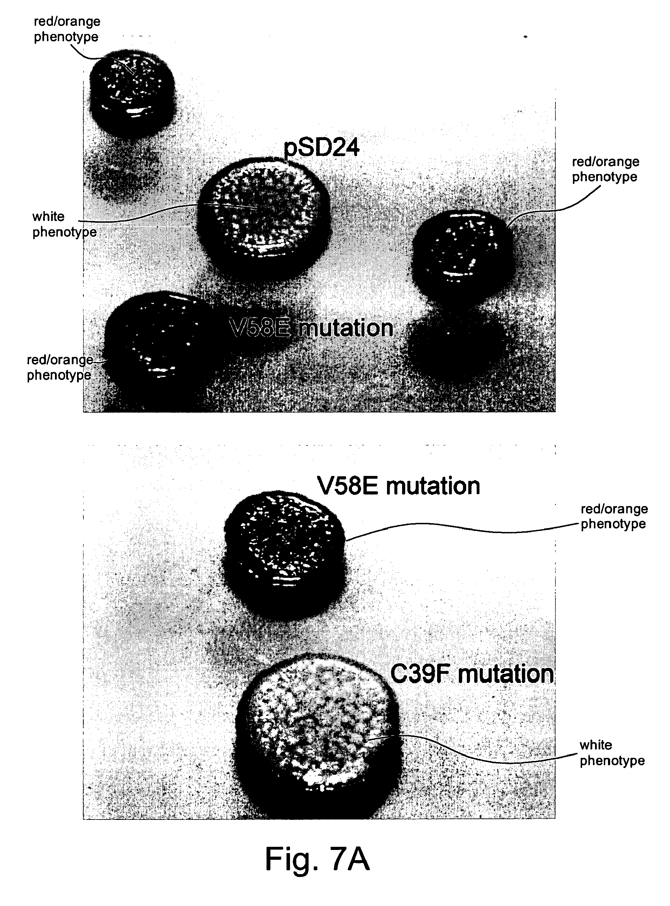

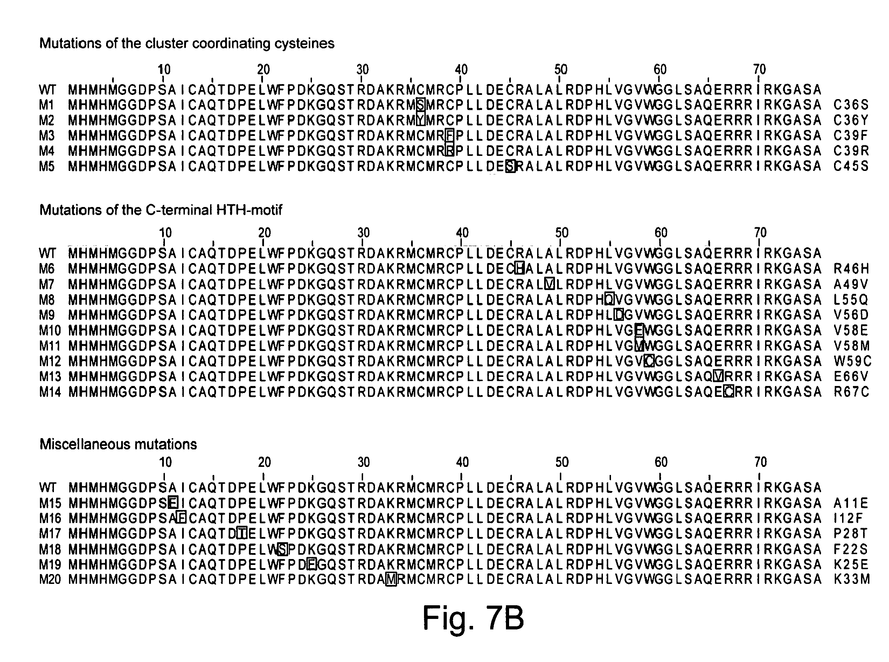

Spontaneous resistance towards the growth inhibitory effect in *M smegmatis* clones expressing WhiBTM4 occurs at a rate of 5x10⁻³ . Applicant created a plasmid library expressing mutated WhiBTM4 proteins and screened for clones with uninhibited growth when expressing proteins harbouring sensitive mutations. Cells transformed with the pSD24WhiBTM4*M* mutant library and plated on 7H10 containing 0.2% acetamide result in an acetamide resistant phenotype at a rate of 1:3. Two major phenotypes can be observed on these plates: approximately 50% of the colonies are relatively large and white resembling wild-type *M smegmatis* colonies and approximately 50% are somewhat smaller colonies with a red to orange colour (Fig. 7A). Colony-PCR of 100 individual clones amplifying the insert of pSD24 and subsequent sequencing of the products shows that approximately 50% of the white colonies have been selected for re-ligated plasmids without insert. Most of the remaining white colonies have mutations resulting in the insertion of stop codons upstream or within the cluster co-ordinating cysteines (not shown). However, two mutants have single amino acid exchanges of the cysteine at position 39 (Fig. 7A/B). All plasmids of the red colonies have mutated WhiBTM4 proteins and approximately 50% of these show single amino acid exchanges resulting in cytotoxicity resistance. These mutations cluster in the C-terminal HTH-motif of WhiBTM4 (Fig. 7B). This part of the WhiB-like proteins has been associated with DNA-binding properties and signal transduction (Soliveri *et al*., 2000). However, applicant notes that 6 of the 17 single amino acid mutations inducing cytotoxicity resistance affect non-cysteine and non-HTH-motif amino acids (Fig. 7B). The role of these amino acids in the function of WhiBTM4 and other WhiB proteins is not clear. Among the orange colonies we found a C45S mutation of the most C-terminal cysteine and C36S/Y mutations. No cytotoxicity resistant clone harbouring mutated C13 (the most N-terminal cysteine) can be recovered from the screen. In WhmD this cysteine was shown to be non-essential for the complementation of a WhmD mutant which might be an explanation for the lack of this mutation in our cytotoxicity resistance screening (Raghunand and Bishai, 2006b).

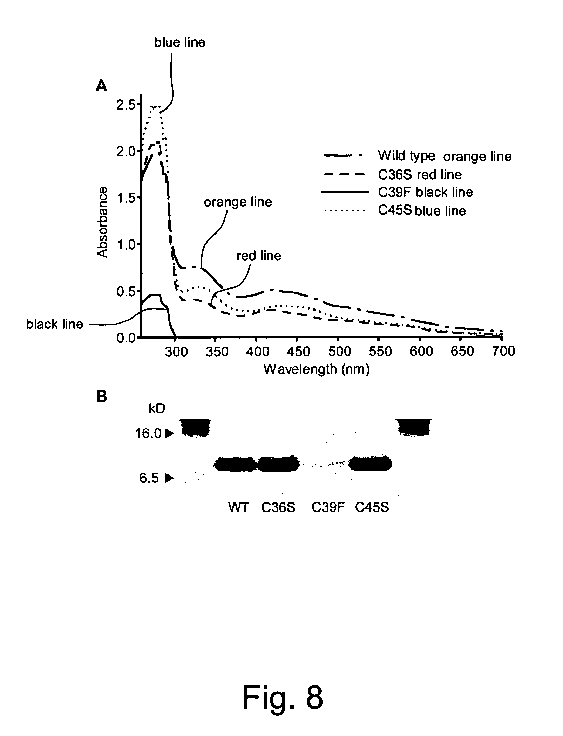

Genes containing the C36S, C39F and C45S exchange mutations are amplified by PCR and cloned into pQE80 for expression and UV-visible absorption spectroscopy. Only small amounts of protein carrying the C39F mutation can be purified and there was no UV-visible absorption indicating the presence of a Fe-S cluster (Fig. 8A/B). This mutation not only seems to inhibit Fe-S cluster assembly but also destabilizes the protein to an extent that prevents sufficient expression and purification indicating the vital role of this amino acid and explaining the white colour of *M smegmatis* clones expressing this mutated gene. Protein destabilization is a common observation in Fe-S proteins carrying sensitive cysteine mutations (Klinge *et al*., 2007). The C45S and C36S mutations allow protein expression and purification comparable to wild-type protein and the eluate of these mutated proteins shows a faint brown colour. UV-visible absorption spectra indicate the presence of a Fe-S cluster with a low A₄₂₃/A₂₈₀ ratio (Fig. 8A) and disassembly of the clusters under atmospheric oxygen is approximately twice as fast compared to wild-type protein (not shown).

Applicant had identified and characterized the bacteriophage derived Fe-S protein WhiBTM4. This is the first description of a viral protein capable of co-ordinating a Fe-S cluster. Since the sequence of WhiBTM4 is highly homologous to the bacterial Fe-S co-ordinating WhiB family of proteins, functional interpretation of this protein requires substantial knowledge of its bacterial counterparts. Though mycobacterial WhiB proteins have been predicted to be transcriptional regulators and Fe-S co-ordinating properties have been shown in some proteins, it is not clear through which mechanism they transmit transcriptional information. The best described mycobacterial WhiB-like protein is WhiB3. This protein has been shown to coordinate an oxygen sensitive [4Fe-4S] cluster with the help of the *M tuberculosis* cysteine desulfurase IscS (Singh *et al*., 2007). A WhiB3 knock-out mutant was sensitive to nutritional stress and grew poorly on agar with a single carbon source. The interaction of WhiB3 with the principal sigma factor RpoV of *M tuberculosis* was shown with the help of a yeast-two-hybrid screen (Steyn *et al*., 2002). However, it is not clear whether WhiB3 modifies promoter specificity of RpoV or functions as an anti-sigma factor. WhiB4 of *M*. *tuberculosis* has recently been shown to co-ordinate a [4Fe-4S] cluster under anaerobic conditions *in vitro* and loss of the cluster leads to disulphide reductase activity of this protein

(Alam *et al*., 2007). In contrast, applicant shows that WhiBTM4 co-ordinates a [2Fe-2S] cluster when kept in an oxygen depleted atmosphere (Fig. 6). It is not possible to rule out that WhiBTM4 may build up a [4Fe-4S] cluster *in vivo,* possibly with the help of a host protein such as IscS. Applicant notes that anaerobic incubation of apoWhiB3 without IscS *in vitro* did not lead to the assembly of a cluster as indicated by the lack of ³⁵S accumulation (Singh *et al*., 2007). Although mycobacterial WhiB proteins have not yet been shown to be redox-responsive transcriptional and post-transcriptional regulators, these functions have been determined for other microbial Fe-S proteins (Green and Paget, 2004). In *E. coli* the [2Fe-2S] co-ordinating SoxR protein is involved in sensing superoxide and nitric-oxide stress, initiates the up-regulation of more than 40 proteins and has DNA- binding capability (Wu *et al*., 1995). FNR, a [4Fe-4S] cluster protein of *E*. *coli* activates the expression of genes that permit reduction of alternative electron acceptors. A C-terminal HTH-motif enables FNR to bind specific DNA sequences (Khoroshilova *et al.,* 1997; Lazazzera *et al*., 1996).

It is an intriguing finding that a viral protein expressed early in the infectious cycle is capable of sensing hypoxia, normoxia and the presence of reducing or oxidizing agents. The benefit for the phage is not clear. It is unlikely that WhiBTM4 undergoes the relatively slow cycle of cluster assembly, loss and re-assembly as part of a redox sensing machinery during the brief presence of the protein in the early stage of phage infection. Recently it was shown that the protein RsmA of *S. coelicolor* binds to and modulates the sigma factor σ_{M}. This interaction was dependent on the presence of a [2Fe-2S] cluster (Gaskell *et al*., 2007). Since sigma factor-binding properties were also shown for a WhiB protein (WhiB3), a possible role for WhiBTM4 and other phage-derived WhiB-like proteins could be the alteration of host sigma factors changing their specificity towards early or late phage promoters. These phage derived anti-sigma factors are well known in other bacteriophages such as T4 where asiA remodels sigma70 of *E. coli* changing its promoter specificity towards phage derived promoter sequences (Orsini *et al*., 1993). In the case of WhiBTM4, cluster loss (or assembly) may cause structural changes in the protein that could lead to the transition from early regulatory phage genes to late structural genes. Thus, WhiB-like proteins of mycobacteriophages could be "time-sensors" rather than redox sensors. However, so far applicant was not able to identify a WhiBTM4-interacting host protein and in other mycobacteriphages the presence of these anti sigma factors has not yet been proven.

Under atmospheric oxygen cluster loss in host derived WhiB proteins such as WhiB4 can be observed over a period of more than 84 hours (Alam *et al*., 2007, 2008). This stands in contrast to WhiBTM4 where disassembly of the cluster is a process of minutes rather than hours (Fig. 5B). This might reflect the short presence of WhiBTM4 during phage infection where a modified protein results in a more rapid cluster loss.

Sequence comparison of WhiBTM4 with the WhiB family proteins of actinomycetes revealed highest homology with 66 amino acids of the C-terminal part of WhiB2/WhmD of *M. tuberculosis* and *M smegmatis* (Fig. 1). This C-terminal part represents the active part of WhmD which is capable of rescuing a WhmD deletion mutant (Raghunand and Bishai, 2006b). WhmD and WhiB2 are important for septum formation and fragmentation and their presence is highly linked with the exponential growth phase (Gomez and Bishai, 2000). Thus it was suggested that they might play a role in the early stages of cell division. The fact that the WhmD knock out mutant could not be substituted by the remaining six WhiB-family proteins in *M smegmatis* indicates a distinct function of this protein (Raghunand and Bishai, 2006b). Applicant's data show that WhiBTM4 leads to down-regulation of WhmD with subsequent filamentation and growth inhibition. Furthermore, applicant could show that proper Fe-S cluster co-ordination is mandatory for this phenotype. It is not clear why mycobacteriophage TM4 affects the transcription of proteins involved in cell division with the help of the early phage protein WhiBTM4. The phage genes responsible for the lysis of the cell wall are situated further upstream in the phage genome and are usually expressed in the late stage of the phage infectioncycle. However, applicant has recently identified another early protein of mycobacteriophage L5 that leads to a similar phenotype when expressed in *M. smegmatis* (Rybniker *et al*., 2008). Interference with cell division and septation seems to be a common observation in the early stage of mycobacteriophage infection.

Eight of the 17 mutations leading to normal bacterial growth during WhiBTM4 expression are situated in the predicted HTH DNA-binding motif represented by the most C-terminal α-helices and an exposed β-turn. This fact strengthens the theory that WhiB proteins transmit their information using this C-terminal structure (Soliveri *et al*., 2000). In contrast to the highly conserved β-turn, the α-helices are less well conserved among the different WhiB proteins and might have different targets explaining the versatile functions of these otherwise highly homologous proteins.

Mycobacteriophage TM4 has a broad host range and is lytic in most of the mycobacteria it infects. In contrast to other mycobacteriophages such as L5 and Bxb1, TM4 infection does not lead to a rapid and global shut-off of host protein synthesis immediately after infection (Ford *et al*., 1998). SDS-PAGE of mycobacterial proteins after infection of the cells with TM4 shows that host gene expression is unchanged throughout the late phase of the cycle which starts approximately 60 minutes after infection (Fig. 3A). This observation is supported by applicants qRT-PCR experiments where 16S rRNA and HSP60 mRNA-levels are stable during TM4 infection. However, WhmD mRNA levels clearly decline. Since WhmD is an essential host-gene and WhiBTM4 expression is growth inhibitory, the protein functions as a moresubtle shut-off protein most likely through a distinct but unknown target.

Applicant shows for the first time that over-expression of a WhiB-like protein in mycobacteria leads to a macroscopically visible colour change of the bacterial colonies. In other organisms such as yeast it is well known that the intracellular accumulation of iron loaded siderophores leads to a similar phenomenon. It is most likely that the observed change in colour presents a non-toxic accumulation of iron bound to WhiBTM4 or another iron binding protein (Lesuisse *et al*., 2005). Thus, applicant concludes that bacterial WhiB family proteins may have an additional function as siderophore-like iron storage proteins. There is major scientific interest in molecules involved in accumulation, detoxification and storage of iron especially in the intracellular pathogens such as *M tuberculosis* that inhabit iron-limited environments. These siderophore-like molecules have recently been identified as interesting drug targets in mycobacteria (Ferreras *et al*., 2005).

The systematic sequencing of more than thirty mycobacteriophages identified a tremendous amount of interesting genetic data spanning a diverse range of gene families such as modified host-derived genes, clearly bacteriophage derived genes and a substantial number of genes with no match in the database (Hatfull *et al*., 2006; Pedulla *et al*., 2003). As with sequence data of any species, the insight into the whole picture of genetic function and gene-interplay can only be provided through the careful analysis of biochemical properties of the proteins encoded by these genes. Here applicant shows that the expression and analysis of a phage derived protein may give information not only on the phage itself, but also on essential bacterial proteins. Particularly the analysis of phage genes that are homologous to but not necessarily identical to host genes is a pioneering way to dissect the function of poorly understood genes of the host.

### Plasmids, primers and bacterial strains

A description of the plasmids, primers and bacterial strains used in this study is given in table S2 and S3. A description of the bacteriophage used in this study is given in table S2.

### Expression of WhibTM4 in M. smegmatis and microscope techniques

WhibTM4 (ORF49) was amplified by PCR using the primer pair 49for and 49rev and TM4-DNA as a template. The product was cloned into BamHI cut pSD24 using the In-Fusion PCR cloning technique (Clontech). After transformation into the E. *coli* Fusion Blue strain the plasmid pSD24WhibTM4 was isolated, sequenced and transformed into *M. smegmatis* mc²155 (see supporting information for full description of strains, plasmids and primers used in this study). Clones were grown on Middlebrook 7H10 agar containing 10 % ADC and 50 µg/ml hygromycin B. For microscopy studies single colonies were picked and grown in 7H9 broth supplemented with 10 % ADC, 0.05% Tween-80 and 50 µg/ml hygromycin B until an OD of 1 at A₆₀₀ was reached, then cells were diluted 1:10 v/v in 7H9 broth containing 0.02 % acetamide. Cells were collected at different time-points, washed in sterile water and stained.

The TB stain kit (Becton Dickinson) was used for Ziehl-Neelsen acid fast staining. For fluorescence microscopy cells were washed in water, the pellets were resuspended in 500 µl SYTO 11 (Molecular Probes) at a final concentration of 5 µM/aqua dest. and incubated at 37°C for 30 min. Cells were then washed several times in water until the supernatant was colorless. 20 µl of cells were mounted on microscope slides in ProLong Gold Antifade Reagent (Invitrogen). Images were acquired with an inverted Olympus IX81 microscope (Olympus) equipped with a F-View II Trigger camera and then analyzed by analySISD software (Soft Imaging Software, Muenster, Germany).

### RT-PCR for the quantification of gene expression during phage infection

*M smegmatis* mc²155 was grown to late log phase in 7H9 medium containing 10 % ADC and 1mM CaCl₂ without Tween-80. The cells were centrifuged and the pellets resuspended in warm 7H9 medium with 1mM CaCl₂ but without ADC. Larger clumps were allowed to settle for five minutes and cells in the supernatant were adjusted to an OD of 0.1 at A₆₀₀ in a total volume of 0.8 litres 7H9 medium with 1mM CaCl₂. (approximately 2 x 10₁₀ cells). Mycobacteriophage TM4 was added at a multiplicity of infection (MOI) of 10 and the cells were shaken slowly at 60 rpm at 37°C. Cells were harvested in 100 ml portions at different time-points by centrifugation for 3 min (4000x g) at 4°C, the pellets were shock frozen and stored at -80°C. Mycobacterial RNA was isolated using the RNeasy MIDI-kit (Qiagen) including the on-column DNAse digestion step. RNA (1 µg) was reverse transcribed with the QuantiTect Reverse Transcription kit (Qiagen). Real-time PCR was performed using the QuantiFast SYBR green PCR kit (Qiagen) on a GeneAmp 5700 sequence detection system (Applied Biosystems). Primers were H27/H28 for the detection of WhiBTM4, H23/H24 for the mycobacterial 16S rRNA, H25/H26 for HSP60 and H31/H32 for the detection of WhmD of *M. smegmatis.* Genomic DNA was used to generate a standard for the mycobacterial genes and pSD24WhiBTM4 to standardize the amount of WhiBTM4.

### Purification of WhibTM4

WhiBTM4 was PCR-amplified using the primer set Q8049 for and Q8049rev and cloned into the expression plasmid pQE80 (Qiagen) in frame with the hexa-histidine tag. E. *coli* BL21 (DE3) cells were transformed with pQE80WhiBTM4 and grown in LB-broth containing 100 µg/ml ampicillin to an OD of 0.6 at A₆₀₀. Expression was induced with 0.5mM IPTG for 2 h at 30°C without shaking. For purification under native conditions, a cell-pellet from a 800 ml culture was resuspended in 20 ml buffer A (200 mM NaCl, 50 mM NaH₂PO4, 30 mM Imidazol, pH 8.0) plus EDTA-free protease inhibitor cocktail (Roche). Cells were disrupted in a FRENCH press (Thermo scientific) at 1250 psi, the soluble lysate was separated by centrifugation at 17.000 x g for 15 min at 4°C and loaded on a Ni₂₊-NTA affinity column (HisTrap FF crude, GE-Helthcare). The column was washed with 15 volumes of buffer A and eluted with buffer B (200 mM NaCl, 50 mM NaH₂PO₄, 500 mM imidazole, pH 8.0) on an ÄKTA-FPLC system using the UNICORN workstation software (GE-Healthcare). For purification under denaturing conditions the lysis/wash-buffer was buffer C (8M urea, 100mM NaH₂PO₄, 10 mM Tris-HCL, 30 mM imidazole, pH 8.0) and elution- buffer D (8M urea, 100mM NaH₂PO₄, 10 mM Tris-HCL, 500 mM imidazole, pH 8.0). All buffers were extensively vacuum degassed and purged with nitrogen. Eluted protein was immediately examined by spectroscopy or frozen in liquid nitrogen for storage at -80°C. Protein concentrations were determined with the Compat-Able BCA protein assay and the Pierce 660 nm protein assay (Thermo scientific). Colorimetric tests were performed with apo WhiBTM4.

### Reconstitution of the Fe-S cluster

Fully oxygenized protein (0.8 mg/ml) purified under denaturing conditions was dialyzed against buffer E (150mM NaCl, 50mM Tris-HCL, 10mM DTT, 100µM FeCl₃, 100µM Na₂S, pH 7.5) for 12 h at 22°C. The dialysis cassette was rinsed with buffer F (200mM NaCl, 50mM Tris-HCL, 10mM DTT, pH 7.5) and dialysed against buffer F at 4°C for 12 h. Again, all buffers were degassed and nitrogen purged. The dialysis steps were performed under strict anaerobic conditions using anaerobic jars that were depleted from oxygen with an Anoxomat system (Mart Microbiology, Netherlands) which evacuates atmospheric oxygen from the jar and refills with an anaerobic gas mixture (nitrogen 85 %, CO₂ 10 %, hydrogen 5%).

### Random mutagenesis of WhibTM4

Low fidelity PCR was performed using the primer pair 49for/49rev and pSD24WhiBTM4 as a template. A 50 µl standard PCR reaction was modified by the addition of 320 µM MgSO4 and 80 µM dGTP (final concentration) and1 µl of the diversify dNTP-mix (Clontech). Cycle conditions were 94°C for 1 minute followed by 25 cycles of 30 sec at 94°C and 1 min at 68°C. Using this setting, the mutational bias is approximately 2.7 mutations per 1000 bp. The PCR products were In-Fusion- cloned into BamHI linearized pSD24 and transformed into E. *coli* Fusion-Blue. Approximately 10₄ individual clones were pooled and plasmids isolated (pSD24WhiBTM4*M*). Approximately 0.5 µg of this randomly mutagenized plasmid library was transformed into *M. smegmatis* mc²155 and clones were plated on 7H10 plates with and without 0.2 % acetamide. Insertions from mutants growing on 0.2% acetamide were sequenced and the sequences were aligned using ClustalW software. To confirm the acetamide resistant phenotype, all mutated WhiBTM4 genes were re-amplified under stringent PCR conditions and the products were cloned into pSD24. The resulting plasmids were transformed into *M. smegmatis* and the transformants plated on 7H10 plates containing 0.2 % acetamide. The inserts of these plasmids were sequenced again as a further proof for a relevant mutation.

### Other analytical techniques

For the quantification of iron by atomic absorption spectrometry (AAS) the WhiBTM4 samples were treated with HNO₃ and diluted in an appropriate amount of ultrapure water. Samples were analysed on an AAnalyst 800 (Perkin Elmer).

UV-Visible absorption spectra were recorded on a Lambda 40 spectrophotometer (Perkin Elmer).

For peptide mass fingerprint (PMF) coomassie stained protein bands were excised from the gel, chopped into cubes and washed three times with acetonitrile-water (1:1). The gel pieces were shrunk with acetonitrile, reswollen in 50 mM NH₄HCO₃ and dried in a speedvac. 10 mM DTT in 50 mM NH₄HCO₃ was added to the dried gel pieces and proteins were reduced for 45 min at 56 °C. To alkylate reduced cysteine residues, the remaining liquid was removed and an equal volume of 50 mM iodoacetamide in 50 mM NH₄HCO₃ was added and the reaction was allowed to proceed for 30 min in the dark. Prior to in gel digestion the gel pieces were washed and dried as above. The gel pieces were reswollen in an ice cold solution of 12.5 ng/µl trypsin (sequencing grade, Promega) in 10 mM NH₄HCO₃. After 45 min on ice, excessive trypsin solution was replaced by 5-20 µl of buffer without enzyme and proteins were digested at 37 °C over night. The digest was stopped by the addition of 5-20 µl 1 % trifluoraceticacid (TFA) in water and peptides were extracted for 30 min at 37 °C. For MALDI-TOF MS of in gel digested proteins 1.0 µl of the extracted peptides was mixed with 1.2 µl of 2.5 mg/ml 2.5-dihydroxybenzoic acid in 0.1 % TFA- acetonitrile (2:1) and spotted onto a 800 µm anchor target (Bruker Daltonics, Bremen, Germany). Positive ion spectra were acquired on a Reflex IV MALDI-TOF mass spectrometer (Bruker Daltonics, Bremen, Germany) in the reflectron mode. A peptide calibration standard (Bruker Daltonics, Bremen, Germany) was used for external calibration of the mass range from m/z 1046 to m/z 3147. The FlexAnalysis postanalysis software was used for optional internal recalibration on trypsin autolysis peaks and the generation of peaklists. Biotools 3.0 (Bruker Daltonics, Bremen, Germany) was used for interpretation of mass spectra with regard to the expected sequence of recombinant 6xHIS-tagged WhiBTM4.

For the determination of protein mass by MALDI-TOF MS samples were desalted and concentrated by micro reversed phase chromatography on C₄-Zip Tips (Millipore). Briefly, the tips were activated in 0.1 % TFA-acetonitrile (1:2), equilibrated in 0.1 % TFA and proteins were adsorbed to the resin by pipetting up and down several times. Salts were removed by extensive washing in 0.1 % TFA and 2.5 µl of a saturated solution of sinapinic acid in 0.1 % TFA-acetonitrile (1:2) were used to elute the tips onto a stainless steel MALDI target. Linear spectra were obtained in the positive ion mode on a Reflex IV MALDI-TOF mass spectrometer (Bruker Daltonics, Bremen, Germany). Protein Calibration Mix I (Bruker Daltonics, Bremen, Germany) was used for external calibration of the spectra in the mass range from 5.7kDa to 17 kDa.

The figures illustrate the present invention, however, without limiting the invention.
**Fig. 1****:** Alignment of WhiBTM4 with *M. smegmatis* (Ms) WhmD and *M. tuberculosis* (Mtb) WhiB2 amino acid sequences. The conserved cysteines are labelled with arrows. The conserved β-turn of the C-terminal HTH-motif is marked at position 117to121.
**Fig. 2****:** Microscopic analysis. *M. smegmatis* cells containing pSD24WhiBTM4 were grown in the presence of 0.02 % acetamide (B, E, F, G, H) or in the absence of acetamide (A, C, D). Ziehl Neelson stained cells (A, B) show a loss of acid fast properties of cells expressing WhiBTM4. After induction for 12 h, cells become highly filamentous and branched (E, F, G, H). SYTO11-stained induced clones show nucleoids throughout the elongated filament suggesting multiple chromosomes per cell (F, H). Magnification of light and fluorescence microscopy: x 1000.
**Fig. 3****:** A. Quantitative RT-PCR of *M. smegmatis* infected with mycobacteriophage TM4 . All data were obtained as triplicates. For better visualization and comparability for each gene at the time-point of highest expression the middle value of the triplicate was defined as 10 (arbitrary unit). All other values for this particular gene were calculated as the ratio of 10. Infection of *M. smegmatis* with TM4 leads to detection of WhiBTM4 RNA after 20 minutes. Presence of WhiBTM4 (oblique) leads to a decline of WhmD (reticular) and HSP60 (horizontal) specific RNA. Values for HSP60 increase during later time-points whereas WhmD levels remain low.
   B. Quantitative RT-PCR of *M. smegmatis* carrying pSD24WhiBTM4. Induction of pSD24WhiBTM4 with different concentrations of acetamide leads to a decrease of WhmD levels which is not seen in cells carrying pSD24 only. Here HSP60 RNA seems to increase independently of the presence of WhiBTM4 (no acetamide: blank; 0.2 % acetamide: horizontal; 0.02 % acetamide: oblique). Error bars indicate the standard error or the mean.
**Fig. 4****:** Coomassie blue stained SDS-PAGE. Five µg of WhiBTM4 were loaded in each lane. Lane 1 and 2, native WhiBTM4; lane 3 and 4 WhiBTM4 purified under denaturing conditions. Samples of lane 2 and 4 were treated with 1mM DTT leading to a loss of the second higher band at approximately 18 kDa and slower migration of the protein. The bands at approximately 6 kDa in lane 1 and 2 are most likely truncated protein samples due to proteolytic digest (note the weak additional band between the 9 and 18 kDa bands (lane 1), indicating the presence of a dimer formed by the truncated form of the protein). Molecular mass is given as determined by MALDI-TOF. All prominent bands were excised and examined with PMF.
**Fig. 5****:** A. UV-visible absorption spectra of WhiBTM4 purified under native conditions (red line) and purified in the presence of 8 M urea (blue line). Numbers indicate the peak at the specified wavelength. This peak pattern is characteristic for proteins carrying a [2Fe-2S] cluster.
   B. Effect of air exposure on the UV-visible absorption spectrum of WhiBTM4. The sample was kept under atmospheric oxygen at 25°C and measured every 10 minutes. The WhiBTM4 cluster is sensitive to oxygen. C. Kinetics of [2Fe-2S] cluster loss determined by the decrease of absorbance at A₄₂₃ upon treatment with different oxidizing and reducing agents. Samples were incubated with 10 mM of DTT (dithiothreitol) (▲), GSSG (oxidized glutathione) (▼) or DTH (dithionite) (◆) and the absorbance recorded every 30 minutes (untreated control: ■). WhiBTM4 senses the presence of reducing an oxidizing agent in-vitro.
**Fig. 6****:** UV-visible absorption spectra of freshly purified WhiBTM4 (orange line), fully oxygenated apoWhiBTM4 (blue line) and reconstituted WhiBTM4 carrying the [2Fe-2S] cluster (red line). The WhiBTM4 [2Fe-2S] cluster can be rebuilt under anaerobic conditions.
   As shown in Fig. 6, the cluster of WhiBTM4 and other WhiB proteins is oxygen labile and can be reconstituted under anaerobic conditions in the presence of FeCl₃ and Na₂S. This process can be visualized in a commonly used photometer as a decrease (loss of cluster) of absorbance at an OD of 423 nm and an increase of absorbance (reconstitution) at an OD of 423 nm. This assay can be used to screen for small molecules that may inhibit either cluster loss or cluster reconstitution. Since proper cluster formation is essential for WhiB protein function and bacterial survival, inhibition of cluster formation by a molecule may be bacteriocidal. Thus, the screen for these molecules may lead to the finding of antimycobacterial substances with a defined target (WhiB proteins). The screening method could be performed in microtitre plates containing purified WhiB protein and a small molecule library of substantial size. One could then search in a photometric plate reader for wells that do not show a decrease in absorbance at 423 nm under an atmosphere rich in oxygen. After the addition of FeCl₃ and Na₂S and the incubation of the plates under anaerobic atmosphere the screen may also lead to wells that do not show an increase of absorbance at 423 nm. These wells may contain molecules inhibiting cluster loss or cluster formation which could then be tested for growth inhibition of *M. smegmatis* and *M. tuberculosis* in a second assay.
**Fig. 7****:** A. Macroscopic appearance of *M. smegmatis* colonies expressing mutated WhiBTM4 species plated on 7H10 agar plates containing 0.2 % acetamide. The red/orange phenotype of the V58E mutation is shown as an example. Cells expressing WhiBTM4 with a C39F mutation have the wild type appearance of cells carrying pSD24 only.
   B. List of the single amino acid mutations allowing WhiBTM4 expression in *M. smegmatis.*
**Fig. 8****:** A. Comparative UV-visible absorption spectra of different cysteine mutations. Eluates of the C45S and C36S mutations show the presence of a Fe-S cluster with a low A₄₂₃/A₂₈₀ ratio.
   B. Samples examined by SDS-PAGE. 5 µl of the FPLC peak eluates were loaded. Total yield of WhiBTM4 with the C39F mutation is low. However, the band shows the proper size excluding cloning errors or the presence of a truncated protein.

### Cited References

Alam, M.S., Garg, S.K., and Agrawal, P. (2007) Molecular function of WhiB4/Rv3681c of Mycobacterium tuberculosis H37Rv: a [4Fe-4S] cluster coordinating protein disulphide reductase. Mol Microbiol 63: 1414-1431.
Alam, M.S., Garg, S.K., and Agrawal, P. (2008) Studies on structural and functional divergence among seven WhiB proteins of Mycobacterium tuberculosis H37Rv. Febs J.
Beinert, H., Holm, R.H., and Munck, E. (1997) Iron-sulfur clusters: nature's modular, multipurpose structures. Science 277: 653-659.
Cole, S.T., Brosch, R., Parkhill, J., Garnier, T., Churcher, C., Harris, D., Gordon, S.V., Eiglmeier, K., Gas, S., Barry, C.E., 3rd, Tekaia, F., Badcock, K., Basham, D., Brown, D., Chillingworth, T., Connor, R., Davies, R., Devlin, K., Feltwell, T., Gentles, S., Hamlin, N., Holroyd, S., Hornsby, T., Jagels, K., Krogh, A., McLean, J., Moule, S., Murphy, L., Oliver, K., Osborne, J., Quail, M.A., Rajandream, M.A., Rogers, J., Rutter, S., Seeger, K., Skelton, J., Squares, R., Squares, S., Sulston, J.E., Taylor, K., Whitehead, S., and Barrell, B.G. (1998) Deciphering the biology of Mycobacterium tuberculosis from the complete genome sequence. Nature 393: 537-544.
Daugelat, S., Kowall, J., Mattow, J., Bumann, D., Winter, R., Hurwitz, R., and Kaufmann, S.H. (2003) The RD1 proteins of Mycobacterium tuberculosis: expression in Mycobacterium smegmatis and biochemical characterization. Microbes Infect 5: 1082-1095.
Ferreras, J.A., Ryu, J.S., Di Lello, F., Tan, D.S., and Quadri, L.E. (2005) Smallmolecule inhibition of siderophore biosynthesis in Mycobacterium tuberculosis and Yersinia pestis. Nat Chem Biol 1: 29-32.
Ford, M.E., Stenstrom, C., Hendrix, R.W., and Hatfull, G.F. (1998) Mycobacteriophage TM4: genome structure and gene expression. Tuber Lung Dis 79: 63-73.
Gaskell, A.A., Crack, J.C., Kelemen, G.H., Hutchings, M.I., and Le Brun, N.E. (2007) RsmA is an anti-sigma factor that modulates its activity through a [2Fe-2S] cluster cofactor. J Biol Chem 282: 31812-31820.
Gomez, J.E., and Bishai, W.R. (2000) whmD is an essential mycobacterial gene required for proper septation and cell division. Proc Natl Acad Sci U S A 97: 8554-8559.
Green, J., and Paget, M.S. (2004) Bacterial redox sensors. Nat Rev Microbiol 2: 954- 966.
Hatfull, G.F., Pedulla, M.L., Jacobs-Sera, D., Cichon, P.M., Foley, A., Ford, M.E., Gonda, R.M., Houtz, J.M., Hryckowian, A.J., Kelchner, V.A., Namburi, S., Pajcini, K.V., Popovich, M.G., Schleicher, D.T., Simanek, B.Z., Smith, A.L., Zdanowicz, G.M., Kumar, V., Peebles, C.L., Jacobs, W.R., Jr., Lawrence, J.G., and Hendrix, R.W. (2006) Exploring the mycobacteriophage metaproteome: phage genomics as an educational platform. PLoS Genet 2: e92.
Jakimowicz, P., Cheesman, M.R., Bishai, W.R., Chater, K.F., Thomson, A.J., and Buttner, M.J. (2005) Evidence that the Streptomyces developmental protein WhiD, a member of the WhiB family, binds a [4Fe-4S] cluster. J Biol Chem 280: 8309-8315.
Khoroshilova, N., Popescu, C., Munck, E., Beinert, H., and Kiley, P.J. (1997) Iron- sulfur cluster disassembly in the FNR protein of Escherichia coli by O2: [4Fe- 4S] to [2Fe-2S] conversion with loss of biological activity. Proc Natl Acad Sci U S A 94: 6087-6092.
Klinge, S., Hirst, J., Maman, J.D., Krude, T., and Pellegrini, L. (2007) An iron-sulfur domain of the eukaryotic primase is essential for RNA primer synthesis. Nat Struct Mol Biol 14: 875-877.
Lazazzera, B.A., Beinert, H., Khoroshilova, N., Kennedy, M.C., and Kiley, P.J. (1996) DNA binding and dimerization of the Fe-S-containing FNR protein from Escherichia coli are regulated by oxygen. J Biol Chem 271: 2762-2768.
Lesuisse, E., Knight, S.A., Courel, M., Santos, R., Camadro, J.M., and Dancis, A. (2005) Genome-wide screen for genes with effects on distinct iron uptake activities in Saccharomyces cerevisiae. Genetics 169: 107-122.
Messerschmidt, A. (2001) Handbook of metalloproteins. Chichester ; New York: Wiley. Morris, P., Marinelli, L.J., Jacobs-Sera, D., Hendrix, R.W., and Hatfull, G.F. (2008) Genomic characterization of mycobacteriophage Giles: evidence for phage acquisition of host DNA by illegitimate recombination. J Bacteriol 190: 2172- 2182.
Morris, R.P., Nguyen, L., Gatfield, J., Visconti, K., Nguyen, K., Schnap-pinger, D., Ehrt, S., Liu, Y., Heifets, L., Pieters, J., Schoolnik, G., and Thompson, C.J. (2005) Ancestral antibiotic resistance in Mycobacterium tuberculosis. Proc Natl Acad Sci U S A 102: 12200-12205.
Orsini, G., Ouhammouch, M., Le Caer, J.P., and Brody, E.N. (1993) The asiA gene of bacteriophage T4 codes for the anti-sigma 70 protein. J Bacteriol 175: 85-93.
Pedulla, M.L., Ford, M.E., Houtz, J.M., Karthikeyan, T., Wadsworth, C., Lewis, J.A., Jacobs-Sera, D., Falbo, J., Gross, J., Pannunzio, N.R., Brucker, W., Kumar, V., Kandasamy, J., Keenan, L., Bardarov, S., Kriakov, J., Lawrence, J.G., Jacobs, W.R., Jr., Hendrix, R.W., and Hatfull, G.F. (2003) Origins of highly mosaic mycobacteriophage genomes. Cell 113: 171-182. Raghunand, T.R., and Bishai, W.R. (2006a) Mycobacterium smegmatis whmD and its homologue Mycobacterium tuberculosis whiB2 are functionally equivalent. Microbiology 152: 2735-2747.
Raghunand, T.R., and Bishai, W.R. (2006b) Mapping essential domains of Mycobacterium smegmatis WhmD: insights into WhiB structure and function. J Bacteriol 188: 6966-6976.
Rouhier, N., Unno, H., Bandyopadhyay, S., Masip, L., Kim, S.K., Hirasawa, M., Gualberto, J.M., Lattard, V., Kusunoki, M., Knaff, D.B., Georgiou, G., Hase, T., Johnson, M.K., and Jacquot, J.P. (2007) Functional, structural, and spectroscopic characterization of a glutathione-ligated [2Fe-2S] cluster in poplar glutaredoxin C1. Proc Natl Acad Sci U S A 104: 7379-7384.
Rybniker, J., Kramme, S., and Small, P.L. (2006) Host range of 14 mycobacteriophages in Mycobacterium ulcerans and seven other mycobacteria including Mycobacterium tuberculosis--application for identification and susceptibility testing. J Med Microbiol 55: 37-42.
Rybniker, J., Plum, G., Robinson, N., Small, P.L., and Hartmann, P. (2008) Identification of three cytotoxic early proteins of mycobacteriophage L5 leading to growth inhibition in Mycobacterium smegmatis. Microbiology 154: 2304- 2314.
Singh, A., Guidry, L., Narasimhulu, K.V., Mai, D., Trombley, J., Redding, K.E., Giles, G.I., Lancaster, J.R., Jr., and Steyn, A.J. (2007) Mycobacterium tuberculosis WhiB3 responds to 02 and nitric oxide via its [4Fe-4S] cluster and is essential for nutrient starvation survival. Proc Natl Acad Sci U S A 104: 11562-11567.
Soliveri, J.A., Gomez, J., Bishai, W.R., and Chater, K.F. (2000) Multiple paralogous genes related to the Streptomyces coelicolor developmental regulatory gene whiB are present in Streptomyces and other actinomycetes. Microbiology 146 ( Pt 2): 333-343.
Steyn, A.J., Collins, D.M., Hondalus, M.K., Jacobs, W.R., Jr., Kawakami, R.P., and Bloom, B.R. (2002) Mycobacterium tuberculosis WhiB3 interacts with RpoV to affect host survival but is dispensable for in vivo growth. Proc Natl Acad Sci U S A 99: 3147-3152.
Van Dessel, W., Van Mellaert, L., Liesegang, H., Raasch, C., De Keersmaeker, S., Geukens, N., Lammertyn, E., Streit, W., and Anne, J. (2005) Complete genomic nucleotide sequence and analysis of the temperate bacteriophage VWB. Virology 331: 325-337.
Wu, J., Dunham, W.R., and Weiss, B. (1995) Overproduction and physical characterization of SoxR, a [2Fe-2S] protein that governs an oxidative response regulon in Escherichia coli. J Biol Chem 270: 10323-10327.

**Table 1. A₄₂₃/A₂₈₀ ratio and total iron content of freshly purified and reconstituted WhiBTM4. Shown are the means of three individual experiments.**

| Sample | A423/A280 | Atoms of iron per WhiBTM4 monomer | Atoms of iron per WhiBTM4 dimer |
|---|---|---|---|
| Freshly purified WhiBTM4 | 0.25 | 0.34 | 0.69 |
| Reconstituted WhiBTM4 | 0.39 | 1.13 | 2.26 |

### Supporting information

**Table S1. Copy numbers of three host genes and WhiBTM4 quantified with RT-PCR after mycobacteriophage TM4 infection of M. smegmatis (A). Copy numbers of genes in M. smegmatis carrying pSD24 or pSD24WhiBTM4 (B). Shown are the means of three different experiments (standard error of the mean in parenthesis).**

| A | | | | | | |
|---|---|---|---|---|---|---|
| | **T = 0** | **T = 20** | **T = 40** | **T = 60** | **T = 90** | **T = 120** |
| **WhiBTM4** | 368.98 | 3.26 x 10⁶ | 1.07 x 10⁶ | 9.36 x 10⁵ | 3.97 x 10⁵ | 4.27 x 10⁵ |
| | (± 83.52) | (± 5.82 x 10⁵) | (± 1.61 x 10⁵) | (± 1.21 x 10⁵) | (± 2.34 x 10⁴) | (± 1.54 x 10⁵) |
| **WhmD** | 8.44 x 10⁴ | 4.66 x 10⁴ | 1.71 x 10⁴ | 1.39 x 10⁴ | 9.01 x 10³ | 6.64 x 10³ |
| | (± 3.29 x 10⁴) | (± 7.5 x 10³) | (± 4.51 x 10³) | (± 1.45 x 10³) | (± 2.05 x 10³) | (± 1.19 x 10³) |
| **HSP60** | 4.17 x 10⁵ | 4.45 x 10⁵ | 1.18 x 10⁵ | 1.95 x 10⁵ | 7.69 x 10⁵ | 8.62 x 10⁵ |
| | (± 8.47 x 10⁴) | (± 7.9 x 10⁴) | (± 4.14 x 10⁴) | (± 2.90 x 10⁴) | (± 1.17 x 10⁵) | (± 3.42 x 10⁴) |
| **16 sRNA** | 1.73 x 10⁸ | 1.57 x 10⁸ | 1.56 x 10⁸ | 1.14 x 10⁸ | 1.35 x 10⁸ | 1.12 x 10⁸ |
| | (± 4.33 x 10⁶) | (± 6.67 x 10⁵) | (± 2.60 x 10⁶) | (± 1.28 x 10⁷) | (±8.72 x 10⁶) | (± 1.58 x 10⁷) |

| B | | | | | | |
|---|---|---|---|---|---|---|
| | **pSDWhiBTM4** | | | **pSD24** | | |
| | ∅ Acetamide | 0.02% Acetamide | 0.2% Acetamide | ∅ Acetamide | 0.02% Acetamide | 0.2% Acetamide |
| **WhiBTM4** | 3.39 x 10⁵ | 1.08 x 10⁸ | 1.28 x 10⁸ | 2.95 | 14.51 | 1.81 |
| | (± 3.27 x 10⁴) | (± 5.17e+006) | (± 1.67e+006) | (± 1.72) | (± 9.13) | (± 1.52) |
| **WhmD** | 2.29 x 10³ | 116.32 | 328 | 672.64 | 697.80 | 958.72 |
| | (± 604.4) | (± 34.15) | (± 34.32) | (± 147.6) | (± 141.4) | (± 165.8) |
| **HSP60** | 1.08 x 10³ | 6.41 x 10³ | 1.10 x 10⁴ | 5.27 x 10³ | 1.17 x 10⁴ | 1.59 x 10⁴ |
| | (± 247) | (± 1.53 x 10³) | (± 1.72 x 10³) | (± 730.3) | (± 1.53 x 10³) | (± 1.57 x 10³) |
| **16 sRNA** | 6.07 x 10⁷ | 5.92 x 10⁷ | 6.53 x 10⁷ | 5.86 x 10⁷ | 5.76 x 10⁷ | 6.03 x 10⁷ |
| | (± 3.18 x 10⁵) | ± 6.69 x 10⁵) | (± 8.02 x 10⁵) | (± 4.33 x 10⁵) | (± 1.01 x 10⁶) | (± 1.05 x 10⁶) |

**Table S2. Strains, bacteriophage and Plasmids used in this document.**

| **Strains, bacteriophage, plasmids** | **Description** | **Reference or source** |
|---|---|---|
| ***Escherichia coli*** | | |
| Fusion blue | *end*A1, *hsd*R17 (r_{K12}-, m_{K12}+), *sup*E44, *thi-*1, *rec*A1, gyrA96, *rel*A1, lac F'[*pro*A⁺B⁺, /*acl^{q}Z*Δ*M15*::Tn*10*(tet^{R})] | Clontech |
| BL21(DE3) | F⁻ *omp*T *gal dcm lon hsd*S_{B}(r_{B}⁻ m_{B}⁻) λ(DE3 [*lacI lacUV5-*T7 gene 1 *ind*1 *sam*7 *nin*5]) | Lab collection |
| ***M. smegmatis* mc²155** | High efficiency transformation mutant | Lab collection |

| **Bacteriophage** | | |
|---|---|---|
| Mycobacteriophage TM4 | Virulent phage of *M. smegmatis.* The NCBI accession number for the full sequence is NC_003387 | Lab collection |

| **Plasmids** | | |
|---|---|---|
| pQE80 | Expression vector carrying the *cis-laqIq* repressor gene | Qiagen |
| pSD24 | Acetamide inducible mycobacterial expression vector | (Daugelat *et al.,* 2003) |
| pSD24WhiBTM4 | pSD24 allowing the expressing of full length WhiBTM4 in *M. smegmatis* | this document |
| pQE80WhiBTM4 | pQE80 allowing the expression of WhiBTM4. The three ATG start codons at the 5' end were deleted for proper fusion with the 6xHIS-tag | this document |

**Table S3. Primers used in this document.**

| **Name** | **Sequence** | **Description** |
|---|---|---|
| 49for | TTTTATCCATGGATCCATGCACATGCACAT | this document |
| 49rev | TCTGCAGCTGGATCCTCATGCCGACGCCCC | this document |
| Q8049for | TCACCATCACGGATCCGGCGGCGACCCGTC | this document |
| Q8049rev | TCAGCTAATTAAGCTTTCATGCCGACGCCC | this document |
| H15 | CCACCACCCGATAAGAGA | this document |
| D26 | TTTATTTGATGCCTGGCAGTC | this document |
| D5 | CGGATAACAATTTCACACAG | this document |
| D6 | GTTCTGAGGTCATTACTGG | this document |
| H27 | CACATGCACATGGGC | this document |
| H28 | CCGACGCCCCTTTG | this document |
| H31 | TGTCATTTTTCGGTTGGTGA | this document |
| H32 | GTCGAACCGGACTACACGAT | this document |
| H25 | CGAGGCGATGGACAAGGT | this document |
| H26 | TACCCTCGGTGAGCTCGA | this document |
| H23 | TGCTACAATGGCCGGTACAAA | this document |
| H24 | CGATTACTAGCGACTCCGACTT | this document |

SEQUENCE LISTING

<110>  Universitaet zu Koeln

<120>  New Therapeutic Concept for the Treatment of Tuberculosis,
especially Pulmonary Tuberculosis

<130>  09.2120.6.ro

<160>  1

<170>  PatentIn version 3.1

<210>  1

<211>  76

<212>  PRT

<213>  Mycobacteriophage TM4

<400>  1

Met His Met His Met Gly Gly Asp Pro Ser Ala Ile Cys Ala Gln Thr
1               5                   10                  15

Asp Pro Glu Leu Trp Phe Pro Asp Lys Gly Gln Ser Thr Arg Asp Ala
            20                  25                  30

Lys Arg Met Cys Met Arg Cys Pro Leu Leu Asp Glu Cys Arg Ala Leu
        35                  40                  45

Ala Leu Arg Asp Pro His Leu Val Gly Val Trp Gly Gly Leu Ser Ala
    50                  55                  60

Gln Glu Arg Arg Arg Ile Arg Lys Gly Ala Ser Ala
65                  70                  75

sequence protocol I

LOCUS NP_569784 76 aa linear PHG 09-OCT-2008
DEFINITION hypothetical protein TM4_gp49 [Mycobacterium phage TM4].
ACCESSION NP_569784
VERSION NP_569784.1 GI:18496934
DBSOURCE REFSEQ: accession NC_003387.1
KEYWORDS .
SOURCE Mycobacterium phage TM4
 ORGANISM Mycobacterium phage TM4
 Viruses; dsDNA viruses, no RNA stage; Caudovirales; Siphoviridae.
REFERENCE 1 (residues 1 to 76)
 AUTHORS Ford,M.E., Stenstrom,C., Hendrix,R.W. and Hatfull,G.F.
 TITLE Mycobacteriophage TM4: genome structure and gene expression
 JOURNAL Tuber. Lung Dis. 79 (2), 63-73 (1998)
 PUBMED 10645443
REFERENCE 2 (residues 1 to 76)
 CONSRTM NCBI Genome Project
 TITLE Direct Submission
 JOURNAL Submitted (19-JAN-2002) National Center for Biotechnology
 Information, NIH, Bethesda, MD 20894, USA
REFERENCE 3 (residues 1 to 76)
 AUTHORS Ford,M.E., Stenstrom,C., Hendrix,R.W. and Hatfull,G.F.
 TITLE Direct Submission
 JOURNAL Submitted (29-MAY-1998) Biological Sciences, University of
 Pittsburgh, Ruskin, Pittsburgh, PA 15260, USA
COMMENT PROVISIONAL REFSEQ: This record has not yet been subject to
 final
 NCBI review. The reference sequence was derived from AAD17616.
 Method: conceptual translation.
FEATURES Location/Qualifiers
 source 1..76
 /organism="Mycobacterium phage TM4"
 /host="Mycobacterium avium"
 /db_xref="taxon:88870"
 /lab_host="Mycobacterium smegmatis"
 Protein 1..76
 /product="hypothetical protein"
 /name="similar to whiB"

/calculated_mol_wt=8360
Region 7..70
/region_name="Whib"
/note="Transcription factor WhiB; pfam02467"
/db_xref="CDD:111373"
CDS 1..76
/locus_tag="TM4_gp49"
/old_locus_tag="TM4_48"
/coded_by="NC_003387.1:33917..34147"
/transl_table=11
/db_xref="GeneID:932296"
ORIGIN
1 mhmhmggdps aicaqtdpel wfpdkgqstr dakrmcmrcp lldecralal rdphlvgvwg
61 glsaqerrri rkgasa

## Claims

1. Use of a pharmacologically active agent being able to inhibit or at least to reduce the expression and/or activity of a (poly-)peptide and/or a protein of a tuberculosis inducing germ, especially the genus *Mycobacterium,* for producing a medicament or pharmaceutical for the prophylactic and/or therapeutic (curative) treatment of tuberculosis, especially pulmonary tuberculosis, wherein the (poly-)peptide and/or protein is associated with or related to the reproduction, especially cell division, of the tuberculosis inducing germ.

2. Use according to Claim 1, wherein the tuberculosis inducing germ is selected from the group of *Mycobacterium* species, especially *Mycobacterium tuberculosis, Mycobacterium bovis, Mycobacterium africanum, Mycobacterium canetti, Mycobacterium microti, Mycobacterium leprae, Mycobacterium avium* and *Mycobacterium kansasii.*

3. Use according to Claim 1 or 2, wherein the (poly-)peptide and/or protein associated with or related to the reproduction, especially cell division, of the tuberculosis inducing germ is associated with or related to the septum formation and/or fragmentation of the tuberculosis inducing germ and/or wherein the (poly-)peptide and/or protein associated with or related to the reproduction, especially cell division, of the tuberculosis inducing germ is a WhiB-like protein, especially a protein of the WhiB-family, preferably WhiB2, more preferably WhiB2 of a species of the genus *Mycobacterium,* even more preferably WhiB2 of *Mycobacterium tuberculosis.*

4. Use according to any of the preceding claims, wherein the pharmacologically active agent is selected form the group of peptides and proteins and/or wherein the active agent is selected from the group of peptides and proteins comprising at least one iron/sulfur-complex and/or having iron/sulfur-coordinating properties and/or wherein the active agent is selected from the group of peptides and proteins comprising at least one DNA-binding motif, especially at least one helix-turn-helix motif.

5. Use according to any of the preceding claims, wherein the pharmacologically active agent is a viral peptide and/or protein, especially a dsDNA-tailed phage peptide and/or protein, preferably a bacteriophage peptide and/or protein, more preferably a mycobacteriophage peptide and/or protein, even more preferably a peptide and/or protein of the bacteriophage TM4.

6. Use according to any of the preceding claims, wherein the pharmacologically active agent is selected from the group of WhiB-like proteins, and/or wherein the pharmacologically active agent is WhiBTM4, especially WhiBTM4 of bacteriophage TM4 or its homologous derivatives and/or its homologous mutations and/or equally acting fragments of WhiBTM4 or combinations thereof.

7. Use of at least one pharmaceutically active agent, especially at least one inhibitor and/or repressor, especially as defined in one of the Claims 4 to 6, for producing a medicament or pharmaceutical for the prophylactic and/or therapeutic (curative) treatment of tuberculosis, especially pulmonary tuberculosis, wherein the active agent suppresses or at least downregulates the activity and/or expression of a gene encoding for a (poly-)peptide and/or protein of a tuberculosis inducing germ, especially of a species of the genus *Mycobacterium,* the (poly-)peptide and/or protein being associated with or related to the reproduction, especially cell division, of the tuberculosis inducing germ.

8. Use of a gene encoding for a (poly-)peptide and/or protein of a tuberculosis inducing germ, especially of a species of the genus *Mycobacterium,* and/or of the corresponding DNA-sequence of the gene and/or of the corresponding RNA-sequence and/or of the encoded (poly-)peptide and/or protein as such, the (poly-)peptide and/or protein being associated with or related to the reproduction, especially cell division, of the tuberculosis inducing germ, for finding and/or providing a pharmaceutically active agent and/or substance especially for the prophylactic and/or therapeutic (curative) treatment of tuberculosis, especially pulmonary tuberculosis, wherein the pharmaceutical active agent inhibits or at least reduces the expression and/or activity of the (poly-)peptide and/or a protein of the tuberculosis inducing germ.

9. Method of identifying and/or retrieving a pharmacologically active agent being able to inhibit or at least to reduce the expression and/or activity of a (poly-)peptide and/or a protein of a tuberculosis inducing germ, wherein the (poly-)peptide and/or protein is associated with or related to the reproduction, especially cell division, of the tuberculosis inducing germ, the method comprising the steps of:
(a) contacting at least one testing molecule with the (poly-)peptide and/or protein being associated with or related to the reproduction of the tuberculosis inducing germ;
(b) detection and/or analysis whether the testing molecule is able to inhibit or at least to reduce the activity of the (poly-)peptide and/or protein being associated with or related to the reproduction of the tuberculosis inducing germ.

10. Method of identifying and/or retrieving a pharmacologically active agent being able to inhibit or at least to reduce the expression and/or activity of a (poly-)peptide and/or a protein of a tuberculosis inducing germ, wherein the (poly-)peptide and/or protein is associated with or related to the reproduction, especially cell division, of the tuberculosis inducing germ, the method comprising the steps of:
(a) incorporating at least one testing substance and/or a DNA-sequence encoding for the testing substance and/or its respective RNA-sequence into a germ being associated with tuberculosis and being able to express the (poly-)peptide and/or the protein of the tuberculosis inducing germ, wherein the (poly-)peptide and/or protein is associated with or related to the reproduction, especially all divison, of the tuberculosis germ, the germ being preferably selected from the group of the genus *Mycobacterium*;
(b) detection and/or analysis whether the testing substance is able to inhibit or at least to reduce the expression and/or activity of the (poly-)peptide and/or protein being associated with or related to the reproduction of the tuberculosis inducing germ.

11. Method of improving the pharmacological properties of a pharmacologically active agent identified by one of the methods according to Claim 9 or 10, the method comprising the steps of
(a) identifying and/or locating the binding site of the pharmacologically active agent to the (poly-)peptide and/or protein being associated with or related to the reproduction of the tuberculosis inducing germ and/or identifying and/or locating the binding site of the pharmacologically active agent to the DNA-Sequence, especially to the gene, and/or to the corresponding RNA-Sequence, encoding for the (poly-)peptide and/or protein being associated with or related to the reproduction of the tuberculosis inducing germ;
(b) modifying the binding site of the pharmacologically active agent, especially via molecular modelling; and
(c) modifying the pharmacologically active agent with the proviso that the specificity and/or affinity and/or avidity of the binding site of pharmacologically active agent is optimized and/or increased with respect to the (poly-)peptide and/or protein being associated with or related to the reproduction of the tuberculosis inducing germ and/or with the proviso that the specificity and/or affinity and/or avidity of the binding site of pharmacologically active agent is optimized and/or increased with respect to the DNA-Sequence, especially to the gene, and/or to the corresponding RNA-Sequence.

12. Method of modification of a pharmacologically active agent identified or improved by one of the methods according to one of Claims 9 to 11, wherein the pharmacologically active agent is further modified in order to attain
(i) a modified active centre, a modified activity spectrum and/or a modified organ specificity, especially lung specificity, and/or
(ii) improved properties with respect to the incorporation and/or uptake properties with respect to the tuberculosis inducing germ and/or
(iii) an improved activity and/or
(iv) a reduced toxicity (an improved therapeutic index) and/or
(v) reduced side effects and/or
(vi) a reduced toxicity (an improved therapeutic index) and/or
(vii) a retarded onset and/or beginning of the therapeutic activity and/or duration of the therapeutic activity and/or
(viii) modified pharmacological parameters, especially resorption, distribution, cell penetration, metabolism and/or excretion, and/or
(iv) modified physicochemical parameters, especially solubility, hygroscopic properties, colour, flavour, smell, stability, application form, and/or
(x) a modified general specificity, especially organ/tissue-specificity,
and/or
(xi) a modified application form and/or application route, especially by
(a) esterification of carboxyl groups and/or
(b) esterification of hydroxyl groups with carbonic acids and/or
(c) esterification of hydroxyl groups, especially to phosphates, pyrophosphates, sulfates and/or hemisuccinates and/or
(d) formation of pharmaceutical acceptable salts and/or
(e) formation of pharmaceutical acceptable complexes and/or
(f) synthesis of pharmacological polymers and/or
(g) introduction of hydrophilic groups and/or
(h) introduction and/or exchanging of isosterical and/or bioisosterical groups and/or
(i) synthesis of homologous compounds and/or
(j) introduction of branched side chains and/or
(k) introduction and/or exchange of substituents in aromatic groups and/or side chains and/or modifying of substituent distribution and/or aubstituent arrangement and/or
(l) conversion of alkyl substituents into cyclic analoga and/or
(m) derivatization of hydroxyl groups into ketals and/or acetals and/or
(n) N-acetylization into amides and/or phenylcarbamates and/or
(o) synthesis of Mannich-bases and/or imines and/or
(p) conversion of ketones and/or aldehydes into Schiff bases, oximes, acetals, ketals, enolesters, oxazolidines, thiozolidines and combinations thereof.
(q) reduction of the molecular size, preferably be deletion and/or exchange of amino acids, however, with the proviso that pharmacologically effective motifs of the pharmacologically active agent are at least essentially not affected.

13. Pharmaceutically active agent, especially for the prophylactic and/or therapeutic (curative) treatment of tuberculosis, especially pulmonary tuberculosis, the pharmacologically active agent being able to inhibit or at least to reduce the expression and/or activity of a (poly-)peptide and/or a protein of a tuberculosis inducing germ, the (poly-)peptide and/or protein being associated with or related to the reproduction, especially cell division, of the tuberculosis inducing germ, wherein the pharmacologically active agent is obtainable by the methods according to Claims 9 to 12.

14. Pharmaceutical composition, especially for the prophylactic and/or therapeutic (curative) treatment of tuberculosis, especially pulmonary tuberculosis, the pharmaceutical composition comprising an efficient amount, especially a pharmacological effective amount, of at least one pharmacologically active agent being able to inhibit or at least to reduce the expression and/or activity of a (poly-)peptide and/or a protein of a tuberculosis inducing germ, the (poly-)peptide and/or protein being associated with or related to the reproduction, especially cell division, of the tuberculosis inducing germ, wherein the pharmacologically active agent is obtainable by the methods according to Claims 9 to 12 and/or wherein the pharmacologically active agent is defined in one of the Claims 4 to 6.

15. Method of treating a human suffering from tuberculosis, especially pulmonary tuberculosis, the method comprising: administering an efficient amount of at least one pharmacologically active agent being able to inhibit or at least to reduce the expression and/or activity of a (poly-)peptide and/or a protein of a tuberculosis inducing germ, the (poly-)peptide and/or protein being associated with or related to the reproduction, especially cell division, of the tuberculosis inducing germ.
